# EUROPÄISCHE PATENTSCHRIFT

(11) **EP 1 066 520 B1**
(45) Veröffentlichungstag und Bekanntmachung des Hinweises auf die Patenterteilung: **21.11.2007**
(21) Anmeldenummer: 99916903.0
(22) Anmeldetag: 01.04.1999
(51) Int. Cl.: G01N 33/52, G01N 33/92, B01J 20/32, G01N 33/48

(54) **WECHSELWIRKUNGEN VON SUBSTANZEN MIT OBERFLÄCHEN AUS AMPHIPHILEN MOLEKÜLEN**
INTERACTIONS BETWEEN SUBSTANCES AND SURFACES THAT ARE MADE UP OF AMPHIPHILIC MOLECULES
INTERACTIONS ENTRE DES SUBSTANCES ET DES SURFACES CONSTITUEES DE MOLECULES AMPHIPHILES

(30) Priorität: 02.04.1998 DE 19814775
(43) Veröffentlichungstag der Anmeldung: 10.01.2001
(73) Patentinhaber: Nimbus Biotechnologie GmbH, 04103 Leipzig (DE)
(72) Erfinder: BAYERL, Thomas, D-97074 Würzburg (DE); LOIDL-STAHLHOFEN, Angelika, D-04129 Leipzig (DE); SCHÖTTNER, Matthias, D-95444 Bayreuth (DE)
(74) Vertreter: Patentanwälte Ruff, Wilhelm, Beier, Dauster & Partner
(86) Internationale Anmeldenummer: PCT/EP1999/002260
(87) Internationale Veröffentlichungsnummer: WO 1999/051984

(56) Entgegenhaltungen:
- DE-A- 4 217 353
- US-A- 5 094 819
- OBRINGER A R ET AL: "Antiphospholipid antibody binding to bilayer-coated glass microspheres" JOURNAL OF IMMUNOLOGICAL METHODS, Bd. 185, Nr. 1, 11. September 1995 (1995-09-11), Seite 81-93 XP004021156 ISSN: 0022-1759
- K MIYAKE, F KITAURA, N MIZUNO: "Phosphatidyl-choline-coated silica as a stationary phase for high-performance liquid chromatography determination of partition coefficients between octanol and water" JOURNAL OF CHROMATOGRAPHY, Bd. 389, 1987, Seiten 47-56, XP002110998 in der Anmeldung erwähnt
- BEIGI F ET AL: "Immobilized-liposome chromatographic analysis of drug partitioning into lipid bilayers" JOURNAL OF CHROMATOGRAPHY A, Bd. 704, Nr. 2, 9. Juni 1995 (1995-06-09), Seite 315-321 XP004023102 ISSN: 0021-9673
- F M LINSEISEN, M HETZER, T BRUMM, T M BAYERL : "Differences in the physical properties of lipid monolayers on a spherical solid support" BIOPHYSICAL JOURNAL, Bd. 72, April 1997 (1997-04), Seiten 1659-1667, XP002110999 in der Anmeldung erwähnt
- H M REINL, T M BAYERL: "Interaction of myelin basic protein with single bilayers on a solid support: an NMR, DSC and polarized infrared ATR study" BIOCHIMICA ET BIOPHYSICA ACTA, Bd. 1151, 1993, Seiten 127-136, XP002111000 in der Anmeldung erwähnt
- C NAUMANN ET AL.: "Phase transition behaviour of single phosphatidylcholine bilayers on a solid support studied by DSC NMR and FT-IR" BIOPHYSICAL JOURNAL, Bd. 63, 1992, Seiten 1314-1319, XP002111001 in der Anmeldung erwähnt
- S ONG, H LIU, X QIU, G BHAT, C PIDGEON: "Membrane partition coefficients chromatographically measured using immobilized artificial membrane surfaces" ANALYTICAL CHEMISTRY, Bd. 67, 1995, Seiten 755-762, XP002111002 in der Anmeldung erwähnt
- L TAMM: "Supported Phospholipid Bilayers" BIOPHYSICAL JOURNAL, Bd. 47, 1985, Seiten 105-113, XP002111003 in der Anmeldung erwähnt
- LUNDQVIST A ET AL: "Chromatography on cells and biomolecular assemblies" JOURNAL OF CHROMATOGRAPHY B: BIOMEDICAL SCIENCES & APPLICATIONS, Bd. 699, Nr. 1-2, 10. Oktober 1997 (1997-10-10), Seite 209-220 XP004094997 ISSN: 0378-4347
- KÖCHY T.; BAYERL T.: 'Lateral diffusioncoefficients of phospholipids in spherical bilayers on a solid support measured by 2H-nuclear-magnetic-resonance relaxation' PHYSICAL REVIEW E Bd. 47, Nr. 3, 1993, Seiten 2109 - 2116
- MANNERS C.N. ET AL: 'Distribution coefficient, a convenient term for the relation of predictable physico-chemical properties to metabolic processes.' XENOBIOTICA Bd. 18, Nr. 3, 1988, Seiten 331 - 350

## Beschreibung

Die Erfindung betrifft zunächst ein Verfahren zur Untersuchung der Wechselwirkungen von Substanzen oder Stoffen mit Oberflächen oder Grenzflächen aus amphiphilen Molekülen sowie die Verwendung eines beschichteten Trägermaterials zur Untersuchung der besagten Wechselwirkungen und einen Kit, welcher zur Durchführung des erfindungsgemäßen Verfahrens geeignet ist.

Die Bestimmung von Bindungskonstanten wasserlöslicher Moleküle an amphiphile Oberflächen bzw. Grenzflächen (insbesondere Lipidmembranen) ist von wachsender Bedeutung für Pharmakologie, Medizin, Biotechnologie und Biochemie. Sie ermöglicht vertiefte Einsichten, wie Moleküle (insbesondere Biomoleküle wie z.B. Peptide, Proteine oder Nukleinsäuren, aber auch biologisch aktive Substanzen und Wirkstoffe) mit biologischen Membranen in Wechselwirkung treten, wie sie innerhalb von Zellen transportiert werden und ihre Signalwirkung entfalten können. Kenntnis von Lipidbindungskonstanten bzw. entsprechender Verteilungskoeffizienten ist unerläßlich für die Entwicklung therapeutisch wirksamer Biomoleküle und anderer, neuartiger Medikamente (Böhm, H.J., Klebe, G., Kubinyi, H., "Wirkstoffdesign", Spektrum, Akad. Verl., 1. Auflage, Heidelberg (1996)).

Im speziellen Fall von Pharmazeutika wird bisher die Näherung eines Verteilungskoeffizienten zwischen dem Alkohol Oktanol und Wasser, der sogenannte K_{ow}-Wert genutzt, um die Lipidaffinität zu charakterisieren (Fujita, T., Iwasa, J., Hansch, C. "A new substituent constant, derived from partition coefficients", J. Am. Chem. Soc. 86, 5175- 5180, (1994)).

Aus der Tendenz einer Verbindung, sich in der unpolareren Alkoholphase aufzuhalten, wird auf ihre Lipidlöslichkeit rückgeschlossen. Biologische Membranen sind jedoch nanostrukturierte, quasi-zweidimensionale Objekte, deren komplexe Eigenschaften durch die Oktanol/Wasser Grenzfläche nur sehr unzureichend imitiert werden können. Als realistischeres Modellsystem sind auch Lipid-Monolayer an der Wasser/Luft Grenzfläche eines Langmuirtroges für die Messung der Lipidbindung von Wirkstoffen eingesetzt worden (Seelibu, A. "Local anestetics and pressure: a comparison of dibucaine binding to lipid monolayers and bilayers", Biochim. Biophys. Acta 899, 196-204 (1997)).

Voraussetzung zur Quantifizierung von Bindungsphänomenen an biologischen Membranen sind genau definierte Lipidmembranmodellsysteme. Nach dem derzeitigen Stand der Technik werden hierfür 3 verschiedene Systeme angewandt: (1) Lipidvesikel, die mit verschiedenen Standardtechniken erzeugt werden können (Mayer, L.D., Hope, M.J., Cullis, P.R. "Vesicles of variable sizes produced by a rapid extrusion procedure", Biochim. Biophys. Acta 858, 161-168 (1986)), (2) planare Lipidschichten (sogenannte Lipidmonolayer), die durch Langmuir-Blodgett Techniken präparierbar sind (Adamson, A.W., Gast, A.P. "Physical Chemistry of Surfaces" Ch. XV., pp. 557-562, Wiley Interscience New York (1997)) und (3) an Festkörpern durch kovalente Bindung immobilisierte Lipidschichten (sogenante immobilisierte Membranen) (Onbo, S., Liu, X, Qiu, X, Bhat, G, Pidgeon, C., "Membrane Partition Coefficients Chromatographically Measured Using Immobilized Artificial Membrane Surfaces", Anal. Chem. 67, 755-762 (1995)).

Alle diese Systeme haben systemspezifische Nachteile. Lipidvesikel sind instabil, neigen insbesondere zu Fusion oder Aggregation und erlauben dadurch keine verläßliche Abschätzung der Oberfläche, die bezüglich den zu untersuchenden Molekülen exponiert wird. Darüber hinaus können die zu untersuchenden Moleküle während der Bindung selbst einen Einfluß auf die Morphologie der Lipidvesikel ausüben (z.B. Induktion einer Vesikelfusion), wodurch die Bindung noch schwerer quantifizierbar wird.

Für Lipidmonolayer besteht ein Problem in der vergleichsweise geringen Oberfläche, die zu den zu untersuchenden Molekülen exponiert werden kann, wodurch die Detektion der Bindung erschwert wird. Ein weiteres Problem der Lipidmonolayer ist ihre Inhomogenität (Bildung von Defekten oder Entmischungen), da Abweichungen von einer homogenen Verteilung der Lipide die Bindung des Moleküls an die Oberfläche beinflussen kann.

Immobilisierte Membranen haben den Nachteil, daß jedes Lipidmolekül kovalent auf der Festkörperoberfläche verankert wird. Dies ist nicht nur ein technisch aufwendiger (und damit teurer) Prozeß, er hat auch im Falle einer mehrkomponentigen immobilisierten Lipidmischung den Nachteil, daß während der Immobilisierung eine Clusterbildung gleichartiger Lipide durch Entmischung stattfinden kann. Dadurch können immobilisierte Membranen im Falle komplexer Lipidmischungen Oberflächenheterogenitäten aufweisen, die das Meßergebnis verfälschen können. Das Problem liegt hier in der kovalenten Bindung an den Festkörper, denn dadurch ist die laterale Diffusivität der Lipide in der Ebene der Membran, ein Charakteristikum jeder natürlichen Membran, völlig unterbunden. Einmal gebildete Heterogenitäten können sich also nicht durch molekulare Diffusion ausgleichen. Die aufwendige Präparationstechnik immobilisierter Membranen verhindert darüber hinaus eine rasche Anpassung der Lipidzusammensetzung auf der Oberfläche an die für das spezielle Experiment wünschenswerte Zusammensetzung.

Die auf diesen Modellsystemen beruhenden Verfahren zur Bestimmung von Lipidbindungskonstanten erfordern deshalb eine Vielzahl von zeitaufwendigen Kontrollexperimenten, um im Experiment genau definierte Mengen an Lipiden und Oberflächen zu exponieren. Die erforderlichen Meßmethoden, um im nächsten Schritt die Bindung des zu untersuchenden Moleküls an die Modellmembran zu quantifizieren, lassen sich in zwei Kategorien einteilen.

Zum einen kann versucht werden, die Bindung zwischen (Bio)molekül und Modellmembran direkt zu erfassen. Dazu bedient man sich z.B. spektroskopischer Meßverfahren wie der Fluoreszenzspektroskopie (Fedoreeva, L.I., Solov'eva, T.F., "Binding of porin with lipopolysaccharide from Yersinia pseudotuberculosis", Biorg. Khim. 21, 17-23 (1995)), dem Circulardichroismus (Terzi, E., Hölzemann, G., Seelig, J., "Selfassociation of beta-amyloid peptide (1-40) in solution and binding to lipid membranes", J. Mol. Biol., 252:5 633-42 (1995)), der Plasmonenresonanzspektroskopie (Salamon, Z., Maclleod, A., Tollin, G., "Surface plasmon resonance spectroscopy as a tool for investigating the biochemical and biophysical properties of membrane protein systems, II Applications to biological systems, Biochim. Biophys. Acta 1331, 131-152 (1997)) oder der Infrarotspektroskopie (Reinl, H.M., Bayerl, T.M. "Interaction of myelin basic protein with single bilayers on a solid support: an NMR, DSC and polarized infrared ATR study", Biochim. Biophys. Acta 1151, 127-136 (1993)). Des weiteren kann aus der Änderung der Reaktionswärme (Isotherme Titrationskalorimetrie (Beschiavili, G., Seelig, J. "Peptide Binding to Lipid Bilayers. Nonclassical Hydrophobic Effect and Membrane-Induced pH Shifts" Biochemistry 31, 10044-10053 (1992)), des Potentials an der Membranoberfläche oder der Oberflächenspannung (Lakhdar-Ghazdal, F., Vigroux, Willson, M., Tocanne, J.F., Périé, J., Faye, J.C. "Interactions between trypanocidal drugs and membrane phospholipids. A surface pressure, surface potential and electrophoretic mobility study", Biochem. Pharmacol. 42(11), 2099-2105 (1991)) auf Bindungsphänomene rückgeschlossen werden. Die Probleme dieser allesamt apparativ sehr aufwendigen Meßmethoden bestehen darin, ein geeignetes, gut detektierbares meßsignal zu finden, welches in eindeutiger Korrelation zur Membranbindung steht und nicht von anderen molekularen Phänomenen überlagert wird. Die rechnerische Auswertung der Meßdaten ist deshalb komplex und kommt meist nicht ohne eine Anzahl von vereinfachenden und nicht immer plausiblen Annahmen aus.

Der zweite Versuchsansatz umgeht die obigen meßtechnischen Probleme teilweise dadurch, daß zur Quantifizierung der Bindung nach erfolgter Inkubation die gebundenen von den nichtgebundenen Molekülen räumlich in unterschiedliche Kompartimente getrennt werden, in denen dann die Konzentrationen der Moleküle separat mittels geeigneter Meßtechnik (z.B. Radioaktivität, Massenspektroskopie, Aktivitätstests, UV/VIS-Spektroskopie) bestimmt werden können. Die Genauigkeit dieses Ansatzes wird jedoch von den oben diskutierten Eigenschaften der Membranmodelle sehr begrenzt. Zur Abtrennung der verfügbaren Lipidvesikelsysteme sind sehr zeitintensive Verfahren wie Gleichgewichtsdialyse, Gelfiltration oder Ultrazentrifugation erforderlich. Zur Beschleunigung der langen Sedimentationszeiten bei der Ultrazentrifugation von Lipidvesikeln erhöht man deren Dichte häufig durch Beladung des inneren Volumens mit Sucrose oder geeigneten Polymeren (Vergeres, G., Ramsden, J.J., "Binding of myristoylated alanine-rich C kinase substrate-related protein (MRP) to vesicular phospholipid membranes", Biochem. J. 330, 5-11 (1998)), was jedoch die Stabilität des Systems verringert und so die Artefaktanfälligkeit der Methode noch beträchtlich erhöht.

Für die Verwendung von Lipidmonolayern bzw. Langmuir-Blodgett-Schichten als Membranmodell können, falls die Trennung in unterschiedliche Kompartimente überhaupt gelingt, nur sehr wenige (und technisch sehr aufwendige) Methoden die erforderliche Empfindlichkeit bei der Detektion der Moleküle liefern. Dies liegt an den, durch die vergleichsweise äußerst geringe Monolayeroberfläche bedingten, extrem geringen Konzentrationen von gebundenen bzw. freien Molekülen. Immobilisierte Membranen haben zwar nicht das Separationsproblem und exponieren auch ausreichend Oberfläche, sind jedoch wegen der oben ausgeführten Nachteile wenig flexibel und bilden kein realistisches Modellsystem einer Membran.

Obringer et al. (Journal of immunological Methods, 185, Nr.1 (1995), S. 81-93) beschreiben das Testen der Immunreaktivität von drei verschiedenen monoklonalen Antiphospholipid-Antikörpern (aPLs), welche unterschiedlich mit Phosphatidylserin (PS) bzw. Cardiolipin (CL) reagieren (Seite 82 in D1). PS bzw. CL stellen hierbei die Epitope dar, mit denen die Antikörper spezifisch wechselwirken. Unspezifische Wechselwirkungen von Molekülen mit Grenzflächen, insbesondere mit Membranen, werden nicht beschrieben.

Die Erfindung stellt sich die Aufgabe, ein neues Verfahren bereit zu stellen, das durch Nutzung festkörperunterstützter Membranen aus Lipiden oder anderen geeigneten amphiphilen Substanzen dem zu untersuchenden Molekül eine weitgehend Biomembran-analoge Oberfläche in einer stabilen, genau definierten Anordnung präsentiert und damit artefaktfreie, reproduzierbare Meßergebnisse ermöglicht. Die Zusammensetzung der festkörperunterstützen Membranen soll dabei mit, im Vergleich zu den immobilisierten Membranen sehr geringem Aufwand variierbar sein und so auch die Untersuchung spezieller Bindungsprobleme an Membranen mit komplexer Zusammensetzung ermöglichen. Die Mobilität der die Membran bilden den Komponenten durch Diffusion soll hierbei trotz der Kopplung an den Festkörper erhalten bleiben, so daß die Diffusionsvorgänge in biologischen Membranen sowie die ultraweiche Grenzfläche zwischen Membran und wäßrigen Kompartiment perfekt imitiert werden. Gleichzeitig soll durch die Anwesenheit des Festkörpers die Separation nach erfolgter Inkubation mit dem zu untersuchenden Molekül sehr einfach zu bewerkstelligen sein.

Diese Aufgabe wird gelöst durch ein Verfahren zur Untersuchung der unspezifischen Wechselwirkung von Substanzen oder Stoffen mit Oberflächen oder Grenzflächen aus amphiphilen Molekülen, die im sogenannten fluiden Zustand auf einem Träger als fester Phase derart fixiert sind, dass sie in ihrer lateralen Diffusion im wesentlichen unbeeinträchtigt sind, wobei
(a) die in einer wässrigen, mobilen Phase vorliegenden zu untersuchenden Substanzen oder Stoffe einerseits und der Träger mit den amphiphilen Molekülen andererseits in Kontakt gebracht werden,
(b) der Träger mit den amphiphilen Molekülen und den mit diesen wechselwirkenden Substanzen oder Stoffen von der mobilen Phase abgetrennt wird,
(c) die Konzentrationen der zu untersuchenden Substanzen oder Stoffe in der mobilen Phase und/oder in der Phase mit dem Träger bestimmt werden und
(d) aus den Konzentrationen die Bindungskonstanten und/oder Verteilungskoeffizienten der Substanzen oder Stoffe bestimmt werden,
wobei die amphiphilen Moleküle Lipide, Lipidderivate, lipidähnliche oder lipidanaloge Substanzen oder Gemische derartiger Substanzen sind.

Die im folgenden Text benutzte Bezeichnung "Lipide" bezeichnet allgemein die Stoffklasse der Lipide (einschließlich der Steroide) sowie lipidanaloge Moleküle und lipidähnliche Amphiphile, die in der Lage sind, durch Selbstorganisationsprozesse in einem geeigneten Lösungsmittel eine Lipiddoppelschicht (Bilayer) oder eine Lipidmonoschicht (Monolayer) zu bilden.

Bilayer sind selbstorganisierte bimolekulare Schichtstrukturen und werden durch Quellung von Lipiden in wäßrigem Milieu bei Temperaturen oberhalb der Phasenübergangstemperatur Tm hergestellt (Sackmann, E."Polymorphism of Lipid/water systems" in "Biophysics" (Hoppe, W., Lohmann, W., Markl, H., Ziegler, H., eds) pp. 425. Springer Verlag Berlin, Heidelberg (1983)). Monolayer sind selbstorganisierte monomolekulare Lipidschichten, die sich spontan an einer Grenzfläche zwischen einem hydrophilen und einem hydrophoben Medium bilden.

Unter "Bindungskonstante" soll im folgenden eine Größe verstanden werden, die die Affinität der Membranoberfläche der festkörperunterstützten Membranen (feste Phase) für eine Substanz beschreibt, die in dem die festkörperunterstützten Membranen umgebenden Medium (mobile Phase) löslich ist. Als Substanz gilt hierbei jede organische oder anorganische Materie, die in der mobilen Phase löslich ist. Unter "Verteilungskoeffizient" soll im folgenden eine Größe verstanden werden, die bei einer gegebenen Ausgangskonzentration der Substanz sowie bei gegebenen Mengen an festkörperunterstützter Membran und mobiler Phase den Konzentrationsquotienten der Substanz zwischen den vorhandenen Kompartimenten festkörperunterstützer Membran und Wasser quantifiziert.

Für die Durchführung des erfindungsgemäßen Verfahrens wird ein anorganischer oder organischer Festkörper verwendet, der auf seiner Oberfläche mit einer monomolekularen (Monolayer) oder bimolekularen (Bilayer) Schicht aus Lipiden, lipidanalogen Molekülen oder lipidähnlichen Amphiphilen nach aus der Literatur bekannten Verfahren beschichtet wird, so daß seine gesamte Oberfläche durch den Monolayer bzw. Bilayer vollständig von dem ihn umgebendem Medium getrennt wird (Tamm, L., "Supported Phospholipid Bilayers" Biophysical. Journal (Biophys. Soe. USA) 47, 105-113 (1985), Linseisen, F.M., Hetzer, M., Brumm, T., Bayerl, T.M. "Differences in the physical properties of lipid monolayers and bilayers on a spherical solid support", Biophysical Journal 72, 1659-1667 (1997)). Die dauerhafte Stabilisierung des Monolayers bzw. Bilayers auf der Oberfläche kann dabei entweder durch nicht-spezifische intermolekulare Kräfte (z.B. van-der-Waals oder elektrostatische Kräfte) oder durch spezifische chemische Bindung an die Oberfläche erfolgen. Durch die Beschichtung entsteht eine biokompatible Festkörperoberfläche, die im folgenden als festkörperunterstützte Membran bezeichnet werden soll. Die physikalischen und physiko-chemischen Eigenschaften festkörperunterstützter Membranen wurden in der Literatur detailliert beschrieben (Naumann, C. et al. "Phase transition behaviour of single phosphatidylcholine bilayers on a solid support studied by DSC NMR and FT-IR" Biophysical Journal 63, 1314-1319 (1992); Bayerl, T.M., Bloom, M. "Physical properties of single phospholipid bilayers adsorbed to microglass beads", Biophysical Journal 58, 357-362 (1990)).

Eine für das erfindungsgemäße Verfahren entscheidende Eigenschaft dieser festkörperunterstützten Membran ist die Tatsache, daß diese als Grenzfläche zu einem wäßrigen Medium von der Seite des Mediums aus praktisch nicht von einer in der Natur vorkommenden Vesikelmembran (z.B. Neurotransmitter-Vesikel) bezüglich ihrer physiko-chemischen und biochemischen Eigenschaften unterschieden werden kann. Dies gilt insbesondere dann, wenn die molekulare Lipidkomposition des Monolayers bzw. Bilayers jener des natürlichen Systems weitgehend angenähert wird. Bindungsvorgänge von in dem wäßrigen Medium gelösten Molekülen an die festkörperunterstützte Membran durch unspezifische Wechselwirkungen werden deshalb weitgehend den analogen Vorgängen im natürlichen System ähneln.

In Anpassung an die experimentellen Erfordernisse kann die Lipidkomposition so gewählt werden, daß die Gesamtladung des beschichteten Trägers neutral oder positiv/negativ geladen ist, wobei eine negative Ladung des beschichteten Trägers im allgemeinen bevorzugt ist.

Die Bestimmung der Lipidbindungskonstante der im wäßrigen Medium gelösten Stoffe (z.B. Peptide, Proteine, Nukleinsäuren oder pharmazeutische Wirkstoffe) an die festkörperunterstützte Membran mit zuvor gewählter und durch die Präparation realisierter Lipidkomposition erfolgt durch eine Titrationsreihe bei konstanter Temperatur. Die Temperatur wird vorteilhafterweise so gewählt, daß alle Lipidkomponenten homogen über die festkörperunterstützte Membran verteilt sind. Dies ist gegeben, wenn die Temperatur oberhalb der Kettenschmelz-Temperatur (im folgenden als Phasenübergangstemperatur (PT) oder Transitionstemperatur bezeichnet) der Lipide liegt. Unter diesen Bedingungen ist die Membran im sogenannten fluiden Zustand, durch den auch alle biologischen Membranen gekennzeichnet sind.

Eine Beeinflussung der Transitionstemperatur kann beispielsweise durch eine Erniedrigung des Druckes erreicht werden. Besonders vorteilhaft ist jedoch eine Beeinflussung der Transitionstemperatur durch eine entsprechend gewählte Zusammensetzung der Lipidkomposition.

Vorteilhafterweise wird die Lipidkomposition derart gewählt, daß die Transitionstemperatur der amphiphilen Moleküle unterhalb der Raumtemperatur liegt. In diesem Fall können die erfindungsgemäßen Verfahren bei Raumtemperatur durchgeführt werden. Dies bringt offensichtliche praktische Vorteile mit sich.

Außerdem kann vorteilhafterweise durch eine Abschätzung sichergestellt werden, daß die bei der Titration angebotene festkörperunterstützte Membranoberfläche immer wesentlich größer ist als die von dem zu untersuchenden Stoff zur Bindung benötigte Oberfläche, da sonst Sättigungseffekte die Messung stören oder verhindern können.

Zur Ausführung der Titration werden entweder (i) unterschiedliche Mengen der auf Bindung zu untersuchenden Stoffe in einheitliche Volumina, immer dieselbe Menge an festkörperunterstützten Membranen enthaltende Gefäße titriert oder (ii) die festkörperunterstützten Membranen werden in unterschiedlichen Konzentrationen in gleiche Volumina, identische Konzentrationen des zu untersuchenden Stoffes enthaltende Gefäße titriert. In beiden Fällen sollte vorteilhafterweise nach der Titration sichergestellt werden (z.B. durch ausreichend lange Inkubationszeit oder durch zusätzliches Schütteln der Gefä-ße), daß sich ein Verteilungsgleichgewicht zwischen membrangebundenen und freien Stoffen in den einzelnen Gefäßen einstellt. Anschließend werden durch geeignete Standardmethoden (Sedimentation, Filtration oder Zentrifugation) die festkörperunterstützten Membranen mit dem an ihnen gebunden Anteil des zu untersuchenden Stoffes aus den wäßrigen Lösungen in den einzelnen Gefäßen abgetrennt. Hierbei ist insbesondere die Abtrennung durch Filtration bevorzugt.

In den verbleibenden wäßrigen Lösungen wird nach Abtrennung der festkörperunterstützten Membranen nun für jede Probe einzeln die in der Lösung verbliebene Konzentration des zu untersuchenden Stoffes bestimmt. Diese Bestimmung kann mittels physikalisch-chemischer oder biochemischer Standardprozeduren erfolgen, die aus den Lehrbüchern bekannt sind und meist zur Grundaustattung eines modernen Labors gehören. Beispiele für solche Methoden sind die optische Spektroskopie, Infrarotspektroskopie, Fluoreszenzspektroskopie sowie chromatographische Techniken.

Aus einer Betrachtung der ermittelten Konzentrationen der Stoffe für jeden Titrationsschritt kann nun mit den aus der Literatur bekannten Verfahren die Lipidbindungskonstante des Stoffes berechnet werden. Damit ermöglicht das Verfahren die rasche Bestimmung einer Lipidbindungskonstanten eines gegebenen, wasserlöslichen Stoffes an eine Membran mit gegebener Lipidkomposition.

Hierbei ist zu beachten, daß die ermittelte Lipidbindungskonstante nur für die bei der Titrationsreihe verwendete Lipidkomposition der festkörperunterstützten Membran gilt. Durch Veränderung dieser Lipidkomposition und wiederholte Messungen läßt sich darüber hinaus die für die maximale bzw. minimale Lipidbindung des gegebenen Stoffes erforderliche optimale Lipidkomposition ermitteln.

Außerdem kann man für eine gegebene Lipidkomposition auch die Lipidbindungskonstante eines interessierenden Stoffes (Substanz A) unter kompetitiven Bedingungen untersuchen, d.h. in Gegenwart eines zweiten Stoffes (Substanz B), der durch eigene Wechselwirkung mit der Membran oder mit der Substanz A oder mit beiden zu einer Veränderung der Lipidbindungskonstante von Substanz A führen kann. Voraussetzung ist hierfür, daß (i) die bei der Titration angebotene Membranoberfläche viel größer als die von Substanz A und Substanz B insgesamt benötigte Oberfläche für eine Membranbindung ist und daß (ii) die Konzentration beider Substanzen im Überstand getrennt ermittelbar ist. Die zweite Bedingung läßt sich beispielsweise durch eine infrarotspektroskopische oder chromatographische Analyse der Überstände erfüllen.

Weiterhin wird von der Erfindung die Verwendung von Trägern, die mit amphiphilen Molekülen in fluider Phase beschichtet sind, zur Untersuchung der Wechselwirkungen von Substanzen oder Stoffen mit Oberflächen oder Grenzflächen aus amphiphilen Molekülen umfaßt, wobei die amphiphilen Moleküle Lipide, Lipidderivate, lipidähnliche oder lipidanaloge Substanzen oder Gemische derartiger Substanzen sind wobei sich aus derart gewonnenen Ergebnissen Rückschlüsse auf die sogenannte Lipophilität ziehen lassen.

Außerdem umfaßt die Erfindung einen Kit gemäß Anspruchen 19-22, der für die Durchführung des erfindungsgemäßen Verfahrens geeignet ist.

Die Vorteile der Verwendung festkörperunterstützter Membranen zur Bestimmung der Lipidbindungskonstanten und/oder Verteilungskoeffizienten von wasserlöslichen Stoffen und Substanzen gegenüber dem Stand der Technik lassen sich wie folgt zusammenfassen:
1. Durch die Verbindung zu Festkörpern, deren Dichte sich von der des wäßrigen Mediums signifikant unterscheiden soll, können die Monolayer bzw. Bilayer unter Verwendung physikalischer Methoden (z.B. Sedimentation, Zentrifugation, Filtration) aus einem wäßrigen Medium leicht abgetrennt werden.
2. Ein weiterer Vorteil ist die (bei bekannter Festkörperoberfläche) exakt quantifizierbare Membranoberfläche pro Volumeneinheit. Dies ist für freie Vesikelmembranen nicht möglich, da diese durch die ihnen immanente Instabilität neben unilamellaren (d.h. nur ein Bilayer) immer auch multilamellare (viele Bilayer geschichtet) Strukturen ausbilden, wodurch die vom wäßrigen Medium aus für das gelöste Molekül tatsächlich zugängliche Membranoberfläche in nicht exakt bestimmbarer Weise reduziert wird. Dies kann zu einer Verfälschung der aus solchen Meßreihen berechneten Lipidbindungskonstanten führen.
3. Weiterhin läßt sich durch gezielte Wahl von Festkörpern mit einem maximierten Oberfläche/Volumen Verhältnis (z.B. nanoporöse Silikate) ein extrem hoher Anteil von festkörperunterstützter Membranoberfläche in einem gegebenen wäßrigen Volumen erhalten, die leicht den erforderlichen Oberflächenbedarf der für die Bindung der im wäßrigen Volumen gelösten Stoffe überschreiten kann.
4. Durch die Kopplung der Membran an den Festkörper wird verhindert, daß durch die Bindung des Stoffes an die Membran morphologische Veränderungen in der Membran induziert werden, die eine Rückkopplung auf das Bindungsverhalten des Stoffes haben können. Ein Beispiel für eine solche Veränderung wäre die durch verschiedene Substanzen beobachtete Induktion einer Fusion von "freien" Membranvesikeln, die bei festkörperunterstützten Membranen ausgeschlossen werden kann.
5. Festkörperunterstützte Membranen stellen einen optimalen Kompromiß zwischen physiologischer Relevanz auf der einen Seite und hoher Stabilität und Reproduzierbarkeit auf der anderen Seite dar. Darüber hinaus ist ihre Lipidzusammensetzung mit geringem technischen Aufwand variierbar und damit sehr gut an das spezifische Meßproblem anpaßbar.

Weiterhin betrifft die Erfindung ein Verfahren zur Bestimmung der Membranaffinität gelöster Stoffe und Substanzen durch Verwendung von festkörperunterstützten Lipidmembranen in der Säulenchromatographie, insbesondere der Hochdruckflüssigkeitschromatographie (HPLC).

Um Lipidaffinitäten von beispielsweise Pharmazeutika zu charakterisieren wird bisher bekanntermaßen die Näherung eines Verteilungskoeffizienten zwischen dem Alkohol Oktanol und Wasser, der sogenannte P_{ow}-Wert genutzt (Fujita, T, Iwasa, J., Hansch, C. "A new substituent constant, _, derived from partition coefficients", J. Am. Chem. Soc, 86, 5175-5180, (1994)). P_{ow} ergibt sich rechnerisch aus dem Konzentrationsquotienten der Zielverbindung in Oktanol und Wasser. Aus der Tendenz einer Verbindung, sich in der unpolareren Alkoholphase aufzuhalten, wird auf ihre Lipidlöslichkeit rückgeschlossen. Der Verteilungskoeffizient für gut wasserlösliche Verbindungen (Wertebereich P_{ow} = -2 bis 4) läßt sich dabei auf klassisch chemischen Wege, d.h. durch Ausschütteln zwischen Oktanol und Wasser und anschließender Konzentrationsbestimmung in den aneinandergrenzenden Phasen ermitteln (Shake-Flask-Method).

Im breiten Wertebereich von P_{ow} = 0-6 wird standardmäßig die Methode der Hochdruckflüssigkeitschromatographie (High-Performance-Liquid-Chromatography; HPLC) an unpolaren Chromatographiematerialien (sog. Reversed-Phase-Sorbentien) eingesetzt. Sie erlaubt es, nach entsprechender Kalibrierung die Verteilungsgleichgewichte zwischen Oktanol und Wasser über Retentionsfaktoren, d.h. über die Verweildauer in den Säulen bis zur Elution abzuschätzen ("Partition Coefficient (n-octanol/water), High Performance Liquid Chromatography (HPLC) Method", OECD Test Guideline 117, 1989). Je größer die Lipidaffinität einer Verbindung, desto stärker tritt sie mit der hydrophoben Chromatographieoberfläche von Reversed-Phase Materialien in Verbindung, d.h. sie wird später eluiert. Der Oktanol-Wasser-Verteilungskoeffizient beschreibt in ausreichender Weise die Lipidaffinität nicht-ionisierter Verbindungen. Er gibt jedoch keine verläßliche Aussage über das Membranbindungsverhalten ionisierter bzw. ionisierbarer Moleküle, wie sie die größte Gruppe pharmazeutisch interessanter Moleküle darstellt. Biologische Membranen sind selbstorganisierte, heterogene Objekte aus verschiedenen amphiphilen Molekülen (hauptsächlich Lipide), deren komplexe Eigenschaften durch die Oktanol/Wasser Grenzfläche nur sehr unzureichend imitiert werden können.

Eine Voraussetzung zur exakten Quantifizierung aller Bindungsphänomene an biologische Membranen sind deshalb genau definierte Membranmodellsysteme. '

Nach dem derzeitigen Stand der Technik werden für die Abschätzung des Membranbindungsverhaltens mittels HPLC-Verfahren zwei verschiedene Membranmodellsysteme als stationäre Phase (Sorbentien) eingesetzt: (1) HPLC-Sorbentien auf Silikatbasis, auf die Lipidschichten bzw. -Konglomerate undefinierter Struktur bis zur Übersättigung der Oberfläche aggregiert wurden (Miyake,K., Kitaura,F., Mizuno,N."Phosphatidylcholine-coated silica as a stationary phase for high-performance liquid chromatography determination of partition coefficients between octanol and water, Journal of Chromatography, 389, 47-56 (1987)) und (2) sogenannte immobilisierte Membranen (IAM's), d.h. Lipidmonoschichten, die auf den Trägerstoffen durch kovalente Bindung fixiert sind (Pidgeon, C. "Immobilized Artificial Membranes", U.S. Patent 4,931,498 (1990); Ong, S., Liu, X., Qiu, X., Bhat, G., Pidgeon, C., "Membrane Partition Coefficients Chromatographically Measured Using Immobilized Artificial Membrane Surfaces", Anal. Chem. 67, 755-762 (1995)).

Beide Systeme haben bezüglich ihrer Anwendbarkeit in der HPLC bzw. im Hinblick auf ihr Potential zur Imitierung einer natürlichen Membran signifikante Nachteile. Die stationäre Phase nach (1) wurde durch Quellen von Dipalmitoylphosphatidylcholin (DPPC) in Anwesenheit des Silikates in einem wäßrigen Medium bei Raumtemperatur erzeugt. Da die Phasenübergangstemperatur von DPPC, bei der das Schmelzen der Alkylketten einsetzt, jedoch bei 41°C liegt, kann durch die beschriebene Art der Herstellung nur eine völlig undefinierte Aggregation von Lipiden in Form von quasi-kristallinen Multischichtvesikeln und Silikat erfolgen, sehr wahrscheinlich verstopfen dabei die Lipidaggregate die Poren des verwendeten Silikatgels und werden so im Gel gegen eine Extraktion durch die mobile Phase stabilisiert. Im übersättigten Zustand sind dann sämtliche Poren des Silikatgels durch die Lipidaggregate verstopft, die der mobilen Phase zugängliche Oberfläche besteht damit im wesentlichen aus (multilamellaren) Schichten aus quasi-kristallinen DPPC und ist somit weitgehend undefiniert bzgl. Gesamtoberfläche und Zahl der Lipidschichten. Es ist offensichtlich, daß Verteilungsgleichgewichte zwischen Wasser und derartig undefinierten Lipidstrukturen lokal unterschiedlich, d.h. heterogen sind und damit eine grundsätzliche Fehlerquelle für die Bestimmung des Verteilungskoeffizienten darstellen.

Ein weiterer gravierender Nachteil für die physiologische Relevanz dieser Methode ist, daß sie nach (1) bei Temperaturen unterhalb der Phasenübergangstemperatur von DPPC ausgeführt wird. Unter diesen Bedingungen befinden sich die Lipidketten in einer all-trans Konformation und sind in einer quasi-kristallinen Ordnung gepackt. Dies ist ein wesentlicher Unterschied zu natürlichen Membranen, die immer in der ungeordneten und hochbeweglichen fluiden Phase sind. Durch die ca. 10% größere Fläche pro Lipid in der fluiden Phase verändert sich aber auch der Verteilungskoeffizient bzw. die Bindungskonstante an die Lipidoberfläche. Damit ist das Resultat der Messung nach (1) nicht unmittelbar auf das Verhalten einer natürlichen Membran übertragbar.

Nach Methode (2) werden die Lipidmoleküle über ihre hydrophoben Fettsäureketten auf entsprechend voraktivierte Chromatographieträger (z.B. Silikatgele) kovalent gebunden. Man erreicht damit eine vollkommen definierte Lipidoberfläche, die aus einer Lipidmonoschicht besteht. Die nun besser definierte Oberfläche wird jedoch mit gravierenden Nachteilen erkauft. Die Präparation von IAM's ist nicht nur ein technisch aufwendiger (und damit teurer) Prozeß, er hat darüber hinaus den Nachteil einer Packungsdichte der Lipide, die nicht durch die intermolekularen Wechselwirkungskräfte zwischen benachbarten Lipiden bestimmt sind, sondern durch die Zahl potentieller Bindungsstellen auf dem Trägermaterial. Damit ist die Lipidpackungsdichte nicht genau definiert und meist geringer als in vergleichbaren natürlichen Membranen. Ein Ausgleich, wie er in fluiden Membranen durch die Diffusion der Lipide in der Membranebene erfolgt, ist hier wegen ihrer kovalenten Fixierung unmöglich.

Für den Fall einer mehrkomponentigen, immobilisierten Lipidmischung existiert der zusätzliche Nachteil, daß es während der Präparation durch Entmischung zur Clusterbildung gleichartiger Lipide kommen kann. Dadurch können entsprechende immobilisierte Membranen aus komplexen Lipidmischungen Oberflächenheterogenitäten aufweisen, die das Meßergebnis verfälschen.

Die aufwendige Präparationstechnik immobilisierter Membranen verhindert darüber,hinaus eine rasche Anpassung der Lipidzusammensetzung auf der Oberfläche an die für das spezielle Experiment wünschenswerte Zusammensetzung.

Die bisher verwendeten HPLC-Sorbentien nach (1) und (2) vermögen also die tatsächlichen physiologischen Gegebenheiten einer natürlichen Membran nur unzureichend zu imitieren und haben dadurch ein beträchtliches Fehlerpotential bei der Übertragung ihrer Ergebnisse auf natürliche Membranen.

Die Erfindung stellt sich daher zusätzlich die Aufgabe, durch Nutzung festkörperunterstützer Schichten aus Lipiden oder anderen geeigneten amphiphilen Substanzen dem zu untersuchenden Molekül eine weitgehend Biomembran-typische, fluide Oberfläche in einer stabilen, geometrisch definierten Anordnung zu präsentieren und damit artefaktfreie, reproduzierbare und für natürliche Membranen relevante Meßergebnisse zu ermöglichen. Die Zusammensetzung der festkörperunterstützen Membranen soll dabei im Vergleich zu den immobilisierten Membranen mit sehr geringem Aufwand veränderbar sein und so auch die Untersuchung spezieller Bindungsprobleme an Membranen mit komplexer Lipidzusammensetzung ermöglichen. Die Mobilität der die Membran bildenden Komponenten durch Diffusion soll trotz der Kopplung an den Festkörper erhalten bleiben, so daß die Diffusionsvorgänge in biologischen Membranen sowie die ultraweichen Grenzflächeneigenschaften zwischen Membran und mobiler Phase perfekt imitiert werden. Durch die Verwendung eines Festkörpers als Trägerstoff, der für die Säulenchromatographie, insbesondere für die HPLC, geeignet ist, soll die Bestimmung von Membran-Wasser-Verteilungsgleichgewichten mit Hilfe dieser effizienten Methoden möglich sein.

Diese Aufgabe der Erfindung wird durch ein Verfahren zur säulenchromatographischen Untersuchung der unspezifischen Wechselwirkung von Substanzen oder Stoffen mit Oberflächen oder Grenzflächen aus amphiphilen Molekülen, die im sogenannten fluiden Zustand auf einem Träger als fester Phase derart fixiert sind, dass die amphiphilen Moleküle in ihrer lateralen Diffusion im wesentlichen unbeeinträchtigt sind, gelöst. Dabei werden
(a) die zu untersuchenden Substanzen oder Stoffe in einer wässrigen, mobilen Phase in die Säule, welche mit dem genannten Träger gefüllt ist, eingebracht,
(b) die zu untersuchenden Substanzen oder Stoffe mit dem Träger in mindestens einem Säulenchromatographie-Lauf, insbesondere einem HPLC-Lauf, in Kontakt gebracht,
(c) die Retentionszeiten der zu untersuchenden Substanzen bestimmt und
(d) aus den Retentionszeiten die Bindungskonstanten und/oder Verteilungskoeffizienten der Substanzen oder Stoffe bestimmt,
wobei die amphiphilen Moleküle lipide, Lipidderivate, lipidähnliche oder lipidanaloge Substanzen oder Gemische derartiger Substanzen sind.

Für die Durchführung des erfindungsgemäßen Verfahrens in der Säulenchromatographie wird ein anorganischer oder organischer Festkörper auf seiner Oberfläche mit einem Monolayer oder einem Bilayer aus Lipiden nach aus der Literatur bekannten Verfahren so beschichtet, daß seine gesamte Oberfläche durch den Monolayer bzw. Bilayer vollständig von dem ihn umgebendem Medium getrennt wird (Tamm, L., "Supported Phospholipid Bilayers" Biophysical. Journal (Biophys. Soc. USA) 47, 105-113 (1985), Naumann, C., et al., "Phase transition behavior of single phosphatidylcholine bilayers an a solid spherical support studied by DSC, NMR and FT-IR", Biophysical Journal 63, 1314-1319 (1992), Linseisen, F.M., Hetzer, M., Brumm, T., Bayerl, T.M. "Differences in the physical properties of lipid monolayers and bilayers on a spherical solid support", Biophysical Journal 72, 1659-1667 (1997)). Durch die angewandten Verfahren wird sichergestellt, daß der besagte Festkörper nur mit einem Bilayer bzw. Monolayer beschichtet ist und alle nicht für die Beschichtung benötigten Lipide wieder entfernt wurden. Die dauerhafte Stabilisierung des Monolayers bzw. Bilayers auf der Oberfläche des Festkörpers erfolgt dabei durch nichtspezifische intermolekulare Kräfte (z.B. van-der-Waals oder elektrostatische Kräfte).

Für die Beschichtung werden Lipide gewählt, deren Tm deutlich unterhalb der Temperaturen liegt, bei der die Messungen (s. unten) ausgeführt werden sollen. Durch die Beschichtung entsteht eine biokompatible Festkörperoberfläche, die im folgenden als festkörperunterstützte Membran bezeichnet werden soll. Die physikalischen und physiko-chemischen Eigenschaften festkörperunterstützter Membranen wurden in der Literatur detailliert beschrieben (Naumann, C., et al., "Phase transition behaviour of single phosphatidylcholine bilayers on a solid support studied by DSC, NMR and FT-IR" Biophysical Journal 63, 1314-1319 (1992); Bayerl, T.M., Bloom, M. "Physical properties of single phospholipid bilayers adsorbed to microglass beads", Biophysical Journal 58, 357-362 (1990)).

Eine für das erfindungsgemäße Verfahren entscheidende Eigenschaft dieser festkörperunterstützten Membranen ist die Tatsache, daß diese als Grenzfläche zu einem wäßrigen Medium von der Seite des Mediums aus praktisch nicht von einer in der Natur vorkommenden Vesikelmembran (z.B. Neurotransmitter-Vesikel) bezüglich ihrer physiko-chemischen und biochemischen Eigenschaften unterschieden werden kann. Dies gilt insbesondere dann, wenn die molekulare Lipidkomposition des Monolayers bzw. Bilayers jener des natürlichen Systems weitgehend angenähert wird. Bindungsvorgänge von in dem wäßrigen Medium gelösten Molekülen an die festkörperunterstützte Membran durch unspezifische Wechselwirkungen werden deshalb weitgehend den analogen Vorgängen im natürlichen System ähneln.

Die Bestimmung des Verteilungskoeffizienten der Substanz an die festkörperunterstützte Membran mit zuvor gewählter und durch die Präparation realisierter Lipidkomposition erfolgt durch das Packen einer definierten Menge festkörperunterstützter Membranen vorzugsweise in eine "High Performance Liquid Chromatography" (HPLC) Säule geeigneter Dimensionen. Die Packungsprozedur einer solchen Säule unterscheidet sich nicht von den aus der chemischen und biochemischen Laborpraxis bekannten Verfahren und ist Personen, die als Labortechniker geschult sind, bekannt.

Vor dem Packen der Säule sollte durch eine Abschätzung sichergestellt werden, daß die zu packende Menge an festkörperunterstützter Membran eine Gesamtmembranoberfläche exponiert, die wesentlich größer ist als jene Fläche, die bei Adsorption einer im nächsten Schritt auf die Säule aufzubringenden Substanz an die festkörperunterstützte Membran maximal in Anspruch genommen werden kann.

Nach dem Packen der Säule wird diese zunächst mit dem für das Trennproblem gewählten Medium (mobile Phase) so lange unter typischen Säulenchromatographieverhältnissen, insbesondere HPLC-Druckverhältnissen, gespült, bis die festkörperunterstützten Membranen in der Säule sich dem Fluß durch optimale Packung angepaßt haben und die durch einen UV-Monitor am Säulenausgang gemessene Basislinie keine signifikanten zeitlichen Veränderungen mehr zeigt (Equilibrierung). Die Temperatur Ts der Säule, sowie die Druckbedingungen im Zuge der Chromatographie müssen so gewählt sein, daß alle in den festkörperunterstützten Membranen enthaltenen Lipide sich im fluiden Zustand befinden. Unter fluiden Zustand der Lipide soll im folgenden der Zustand geschmolzener Fettsäure -bzw.

Alkylketten der Lipide verstanden werden, der allgemein dann erreicht ist, wenn Ts größer als die Phasenübergangstemperatur Tm der Lipide ist. Unter diesen Bedingungen sind die Lipide im Monolayer bzw. im Bilayer hochbeweglich und können sich durch laterale Diffusion über die Membranoberfläche bewegen (Linseisen, F.M., Hetzer, M., Brumm, T., Bayerl, T.M. "Differences in the physical properties of lipid monolayers and bilayers on a spherical solid support", Biophysical Journal 72, 1659-1667 (1997)), wodurch eine homogene Verteilung der Lipidkomponenten über die Membranoberfläche erreicht wird.

Nach erfolgter Equilibrierung wird auf die Säule, insbesondere die HPLC-Säule, eine definierte Menge der zu untersuchenden Substanz aufgebracht. Bei konstanten, für HPLC typischen Druckverhältnissen wird nun die Retentionszeit gemessen, also die Zeit, die vom Aufbringen bis zur Elution der Substanz vergeht (Zeit tR). Diese Retentionszeit ist ein Maß für die Stärke der Wechselwirkung zwischen Substanz und fester Phase. Je stärker die Wechselwirkung, je größer die Retentionszeit bei gegebener Flußrate der mobilen Phase bzw. des Säulendrucks.

Zur Kalibrierung der Säulenparameter wird vorteilhafterweise anschließend Referenzsubstanz auf die Säule aufgebracht und wiederum die Zeit bis zu ihrer Elution gemessen (Zeit t0). Diese Referenzsubstanz wird hierbei unter dem Gesichtspunkt minimaler bzw. vernachlässigbarer Affinität für die festkörperunterstützte Membranoberfläche ausgewählt. Unter diesen Bedingungen ist t0 im wesentlichen vom sogenannten Totvolumen abhängig, welches durch die Länge der Kapillare nach dem Einspritzblock, von der Geometrie der Packung, der Porosität des Trägermaterials sowie von der Flußrate bzw. Säulendruck abhängig ist.

Aus den Zeiten tR und tO wird nach einer Gleichung, die Stand der Technik ist, der Verteilungskoeffizient berechnet. ("Partition Coefficient (n-octanol/water), High Performance Liquid Chromatography (HPLC) Method", OECD Test Guideline 117, (1989); Miyake, K., Kitaura,F. Mizuno, N. "Phosphatidylcholine-coated silica as a stationary phase for high-performance liquid chromatography determination of partition coefficients between octanol and water, Journal of Chromatography, 389, 47-56 (1987)). Mit weiteren Gleichungen nach dem Stand der Technik läßt sich mit diesen Werten auch die Bindungskonstante berechnen.

Das erfindungsgemäße Verfahren beruht auf der Verwendung von fluiden festkörperunterstützten Membranen, die mit einem Bilayer bzw. Monolayer beschichtet sind. Dadurch ist (a) die zur Verfügung stehende Membranoberfläche durch die Oberfläche des Trägermaterials genau definiert, (b) die Membran äußerst stabil gegen eine teilweise Ablösung von Lipiden durch die mechanischen Kräfte infolge der hohen Flußgeschwindigkeiten der mobilen Phase in der Säule, (c) die festkörperunterstützte Membran durch den fluiden Phasenzustand der Lipidschicht und den daraus resultierenden Eigenschaften einer natürlichen Membranoberfläche sehr ähnlich sowie (d) die Lipidzusammensetzung der festkörperunterstützten Membran mit geringen Aufwand an die speziellen Versuchsbedingungen anpaßbar.

Punkt (a) ist von großer Bedeutung, da in den im Stand der Technik unter (1) beschriebenen Versuchen gezeigt wurde, daß eine lineare Korrelation zwischen der Retentionszeit tR und dem in vielen technischen Anwendungen genutzten Oktanol/Wasser Verteilungskoeffizienten P_{ow} nur oberhalb eines kritischen Verhältnisses von Oberfläche des Trägermaterials zu der zur Beschichtung verwendeten Lipidmenge existiert. Bei (1) entspricht dieses kritische Verhältnis vermutlich einer vollständigen Verstopfung aller Poren des Silikatgels mit quasi-kristallinen DPPC-Aggregaten. Im erfindungsgemäßen Verfahren entspricht das kritische Verhältnis genau der Situation, wenn die Gesamtoberfläche des Trägermaterials gleich der gesamten Bilayer- bzw. Monolayerfläche ist, die zur Beschichtung verwendet wurde. Unter diesen Bedingungen ist eine direkte Wechselwirkung der Substanz mit der Oberfläche des Trägers nicht mehr möglich; die Werte für Verteilungskoeffizient und Bindungskonstante werden nur durch die Wechselwirkung an der Grenzfläche Membran / mobile Phase bestimmt. Während dieser Punkt für jede neu gepackte Säule nach dem im Stand der Technik unter (1) beschriebenen Verfahren erst bestimmt werden muß, sind die optimalen Bedingungen nach dem hier vorgestellten Verfahren von Anfang an gegeben. Eine Überladung der Säule mit Lipid, wie in (1) teilweise praktiziert, führt zwar relativ sicher zu einer vollständigen Abdeckung der Oberfläche des Trägermaterials mit Lipidaggregaten, resultiert aber gleichzeitig in einer undefinierten Oberfläche. Außerdem können die überflüssigen Lipide durch die mechanische Kraft des Flusses der mobilen Phase langsam vom Träger gelöst und aus der Säule herausgespült werden. Damit wäre die zur Verfügung stehende Membranoberfläche zeitlichen Veränderungen unterworfen, was eine bedeutende Fehlerquelle darstellen kann.

Gegenüber denen im Stand der Technik unter (2) beschriebenen IAM's haben die festkörperunterstützten Bilayer bzw. Monolayer ebenfalls entscheidende Vorteile. Bei IAMs sind die Lipide mit dem hydrophoben Ende kovalent an den Träger gebunden, damit ist eine laterale Bewegung der Lipide durch Diffusion, wie sie für eine fluide Membran typisch ist, völlig unterbunden. Die Fläche pro Lipid wird bei IAMs nicht primär durch die intermolekularen Wechselwirkungen zwischen benachbarten Lipiden bestimmt, sondern durch die Zahl der auf der Trägeroberfläche vorhandenen kovalenten Bindungsplätze für Lipide. Dadurch sind festkörperunterstützte Bilayer dichter gepackt als IAM' s und können zudem durch laterale Diffusion jegliche Exposition von Trägeroberfläche zur mobilen Phase verhindern. Verfälschungen der Ergebnisse sind mit IAM`s insbesondere auch dann zu erwarten, wenn die Substanz hydrophobe Anteile aufweist, die dazu tendieren, sich in die hydrophoben Bereiche der Membran einzulagern.

Weiterhin ist ein Material für die Säulenchromatographie, insbesondere für die Hochdruckflüssigkeitschromatographie, welches aus einem vorzugsweise druckstabilen Träger besteht, der mit amphiphilen Molekülen beschichtet ist, die sich in fluider Phase befinden beschrieben. Im Falle der Lagerung oder des Transportes dieses Materials kann die Beschichtung des Trägers jedoch auch vorübergehend in eine andere Phase überführt werden. Bezüglich des Trägermaterials sind sphärische, poröse Silikatstrukturen bevorzugt, beispielsweise Silikatgele mit 10 µm oder 30 µm Kugeldurchmesser und 400 nm Porengröße.

Schließlich wird eine mit entsprechendem Material gepackte Säule für die Chromatographie beschrieben. Vorzugsweise handelt es sich hierbei um eine HPLC-Säule. Es werden jedoch auch andere Chromatographiesäulen, wie z.B. FPLC (fast performance liquid chromatography)-Säulen umfaßt.

Die beschriebenen Merkmale und weitere Merkmale der Erfindung ergeben sich aus der nachfolgenden Beschreibung von Beispielen in Verbindung mit den Unteransprüchen. Hierbei können die einzelnen Merkmale jeweils für sich oder zu mehreren in Kombination miteinander verwirklicht sein.

Die folgenden Beispiele stellen spezielle Applikationen des Verfahrens dar, ohne jedoch seine allgemeine Anwendbarkeit einzuschränken.

### Beispiele

### 1. Bestimmung der Lipidbindungskonstante eines vollständig aufgereingten Modellproteins an Lipidmembranen

Ein Gemisch aus 10 mol% anionisch geladenem 1,2-Dimyristoyl-sn-glycero-3-phosphatidylglycerol (DMPG), 89,97 mol% zwitterionischem Lezithin 1,2-Dielaidoyl-sn-3-glycero-3 phosphocholine (DEPC) und 0,03 mol% Fluoreszenzmarker 1-Hexadecanoyl-2-(1-pyrenedecanoyl)-sn-glycero-3-phosphocholine wird in Chloroform suspendiert und das Lösungsmittel vollständig im Vakuum entfernt. Die Lipide werden anschließend im wäßrigen Puffer (pH 7.4) dispergiert und durch Ultraschallbehandlung kleine, unilamellare Vesikel erzeugt. Durch Versetzen mit sphärischen, porösen Silikatstrukturen (Silikatgel mit 30 µm Kugeldurchmesser und 400 nm Porengröße) bei Temperaturen größer als die PT der Mischung entsteht der entsprechende festkörperunterstütze Lipidbilayer durch spontane Fusion der Vesikel auf der Silikatoberfläche. Überschüssige Lipidvesikel werden durch Sedimentation und Abtrennung des Überstandes entfernt. Die Konsistenz der festkörperunterstützten Membran - im folgenden als Membran A bezeichnet - wird mit Hilfe von Fluoreszenzspektroskopie sichergestellt und die Zusammensetzung der Lipidschicht durch Protonen-NMR-Spektroskopie nachgewiesen.

Membran A wird in Puffer A (Tris/HCl pH 7.4) überführt und auf eine Konzentration von 0,1 g/ml eingestellt (entspricht in etwa 5 µmol Lipidbilayer/ml).

Parallel dazu wird eine Stammlösung des vorgereinigten Modellenzyms Cytochrom C [4 mg/ml) ebenfalls in Puffer A angesetzt und für die Tirationsreihe jeweils 100 µl große Doppelproben in 1 ml große Reaktionsgefäße pipettiert. Nach Zugabe unterschiedlicher Mengen der festkörperunterstützen Membran A (20-300 µl) und jeweiligem Einstellen des Reaktionsvolumens auf 1ml mit Puffer A werden die Gefäße eine Stunde bei Raumtemperatur (Bilayer im fluiden Zustand) unter leichtem Schütteln inkubiert. Die feste, biokompatible Oberfläche kann im Anschluß durch eine kurze niedertourige Zentrifugation (5000 x g; Dauer 10 min) vom Überstand getrennt werden.

Die Konzentration an freiem Cytochrom C im Überstand jeder Probe der Titrationsreihe (insgesamt 15 Doppelproben) wird durch UV/Vis-Absorptionsmessungen bei 280 und 410 nm bestimmt. Entsprechende Auswertung der Datenreihe ergibt die Lipidbindungskonstante bzw. den Kp-Wert von 0,325 mM. Die ermittelten Konstanten korrelieren sehr gut mit Literaturdaten herkömmlicher Bindungsstudien (Heimburg, T., Marsh, D., "Protein Surface-Distribution and Protein-Protein Interactions in the Binding of Pheripheral Proteins to Charged Lipid Membranes", Biophysical Journal 68, 536-546 (1995)).

### 2. Bestimmung der Lipidbindungskonstante eines grob vorgereinigten Proteins an Lipidmembranen

Für dieses Beispiel wird zusätzlich ein festkörperunterstützter Lipidbilayer mit einer Lipidkomposition aus 20 mol% 1-Palmitoyl-2-oleoyl-sn-3-gylcero-3-phosphoserine (POPS), 79,97 mol% 1,2-Dielaidoyl-sn-3-glycero-3-phosphocholine (DEPC) und 0,03 mol% Fluoreszenzmarker 1-Hexadecanoyl-2-(1-pyrenedecanoyl)-sn-glycero-3-phosphocholine nach im Beispiel 1 beschriebenen Verfahren auf einer identischen porösen Silikatoberfläche präpariert. Die Integrität bzw. Vollständigkeit der Bilayerbeschichtung wird durch Fluoreszenzspektroskopie und die Zusammensetzung der Lipidschicht durch Protonen-NMR-Spektroskopie nachgewiesen.

Die so erhaltene festkörperunterstütze Membran aus einer binären Lipidkomposition wird in Puffer B (10 mM Tris/HCl pH 7.4, 1 mM EDTA + protease-Inhibitor-Cocktail) dispergiert und im folgenden als Membran B bezeichnet.

Als Bindungspartner wird das aktinvernetzende Gehirnprotein MRP (Hartwig, J. H., Thelen M., Rosen, A., Janmey, P.A., Nairn, A.C., Aderem, A.A. "MARCKS is an actin filament crosslinking protein regulated by protein kinase C and calcium calmodulin"; Nature 356, 618-622 (1992)) untersucht, welches eine Myristoylseitenkette trägt. MRP bindet nach Literaturangaben (Taniguchi, H. , Manenti, S., "Interaction of myristoylated alanine-rich protein kinase C substrate (MARCKS) with membrane phospholipids", J. Biol. Chem. 268 (14), 9960-9963 (1993)) mit hoher Affinität an das bei pH 7.4 anionische Phospholipid Phosphatidylserin. Eine Lösung konstanter MRP-Konzentration (vorgereinigtes Protein) in Puffer B wird in Dreifachproben in verschiedenen Reaktionsgefäßen vorgelegt und mit unterschiedlichen Mengen von Membran A (Titrationsreihe A) und analog mit Membran B versetzt. Inkubation und Abtrennung der festkörperunterstützten Membranen erfolgt wie in Beispiel 1 beschrieben.

Von den verbleibenden Überständen werden Aliquots mit Hilfe von SDS-Polyacrylamidgelelektrophorese (SDS-PAGE), einem biochemischen Standardverfahren, untersucht. Die zugehörige Bande für das MRP-Protein wird jeweils mit Hilfe eines Densitometers quantifiziert und die Densitometerwerte als Konzentrationswerte des MRP interpretiert.

Die anschließende Berechnung der Lipidbindungskonstante nach gängigem Verfahren zeigt für den neutralen Lipidbilayer aus Titrationsreihe A keine MRP-Bindung. In Titrationsreihe B ergeben sich exakt reproduzierbare Bindungskonstanten (Fehler < 5%) an eine Bilayermembran, die zusätzlich 20% des bei pH 7.4 anionischen POPS enthält. Damit ist die aus der Literatur bekannte Affinität von MRP für Phosphatidylserin durch Messungen nach dem erfindungsgemäßen Verfahren bestätigt worden und darüber hinaus eine exakte Bindungskonstante für die gegebene Lipidkomposition bestimmt worden. Die Daten sind schnell und einfach bestimmbar und decken sich mit Meßergebnissen herkömmlicher, experimentell wesentlich aufwendigerer Verfahren (Vergeres, G., Ramsden, J.J. "Binding of myristoylated alanine-rich C kinase substrate-related protein (MRP) to vesicular phospholipid membranes", Biochem. J. 330, 5-11 (1998)).

### 3. Bestimmung der Lipidbindongsionstante eines Pharmazeutikums

Das Verfahren zur Bestimmung von Lipidbindungskonstanten mit Hilfe fester, biokompatibler Oberflächen ist auch für Pharmazeutika anwendbar. Dies wird hier am Beispiel des Lokalanästhetikums Tetracain demonstriert. Zur Untersuchung des Verteilungskoeffizienten dieses Pharmazeutikums zwischen den Phasen Lipid und Wasser werden, wie in Beispiel 1 beschrieben, poröse Silikatstrukturen mit einer ladungsneutralen Membran aus 99,97 mol% zwitterionischem Lezithin 1,2-Dielaidoyl-sn-3-glycero-3-phosphocholine (DEPC) und 0,03 mol% Fluoreszenzmarker 1-Hexadecanoyl-2-(1-pyrenedecanoyl)-sn-glycero-3-phosphocholine beschichtet (im folgenden als Membran C bezeichnet).

Der zu untersuchende Wirkstoff Tetracain wird in Doppelproben in wässriger Lösung (Puffer A) in konstanter Konzentration von 25 µM vorgelegt und mit unterschiedlichen Mengen an Membran C versetzt (Konzentrationsbereich des Lipids 5*10⁻⁵-2*10⁻³ mol/l). Das Gemisch wird lange genug inkubiert, um die Verteilung des Gleichgewichts zu gewährleisten. Anschließend wird die feste, biokompatible Oberfläche durch Zentrifugation oder Filtration aus der Lösung entfernt. Die Konzentration an freiem Wirkstoff im Überstand kann bei der UV-aktiven Verbindung Tetracain schnell mit Hilfe der UV/Vis-Spektroskopie (Detektion bei 304 nm) erfolgen. Alternativ dazu können die verbleibenden lipidfreien Überstände mit chromatographischen Methoden (HPLC, Gaschromatographie etc.) analysiert werden. Dies bietet wiederum die Option, mehrere Wirkstoffe simultan zu untersuchen.

Der ermittelte Verteilungskoeffizient für Tetracain zwischen der festkörperunterstützen Membran C und dem wäßrigen Kompartiment beim physiologisch relevanten pH-Wert 7.4 beträgt 1,4. Die Bindungskonstanten werden nach gängigen Verfahren errechnet.

### 4. Präparation einer HPLC-Säule aus festkörperunterstützten Membranen

Ein natürliches Gemisch aus Ei-Lezithin und 0,03 mol% Fluoreszenzmarker 1-Hexadecanoyl-2-(1-pyrenedecanoyl)-sn-glycero-3-phosphocholine wird in Chloroform suspendiert und das Lösungsmittel vollständig im Vakuum entfernt. Die Lipide werden anschließend im wäßrigen Puffer (pH 7.4) dispergiert und durch Ultraschallbehandlung kleine, unilamellare Vesikel erzeugt. Durch Versetzen mit sphärischen, porösen Silikatstrukturen (Silikatgel mit 10 µm Kugeldurchmesser und 400 nm Porengröße) bei Temperaturen größer als die Tm der Lipide entsteht der entsprechende festkörperunterstützte Lipidbilayer durch spontane Fusion der Vesikel auf der Silikatoberfläche. Überschüssige Lipidvesikel werden durch Sedimentation und Abtrennung des Überstandes entfernt. Die Konsistenz der festkörperunterstützten Bilayer - im folgenden als Membran A1 bezeichnet - wird mit Hilfe von Fluoreszenzspektroskopie und Analytik des Gesamt-Kohlenstoffgehaltes sichergestellt und die Zusammensetzung der Lipidschicht durch HPLC-Analytik nachgewiesen.

Die Präparation festkörperunterstützter Monolayer (im folgenden als Membran A2 bezeichnet) erfolgt mit der oben verwendeten Lipidmischung nach (Linseisen, F.M., Hetzer, M., Brumm, T., Bayerl, T.M. "Differences in the physical properties of lipid monolayers and bilayers on a spherical solid support", Biophysical Journal 72, 1659-1667 (1997)).

Membran A1 wird in Puffer A (Tris/HCl pH 7.4) überführt und mit etablierten Verfahren sowohl in eine analytische HPLC-Säule der Dimension 150 x 4 mm als auch in die Vorsäule (Dimension 50 x 10 mm) gepackt (Veronika R. Meyer "Praxis der Hochleistungs-Flüssigchromatographie", Verlag M. Diesterweg GmbH, 5. Auflage, Frankfurt am Main, (1988)). Die Vorsäule wird zwischen Pumpe und Injektorblock geschaltet, die eigentliche analytische Säule nach dem Injektorblock. Das Packen von Membran A2 in identische HPLC-Säulen erfolgt analog.

### 5. Kalibrierung einer HPLC-Säule aus festkörperunterstütsten Membranen

Zum "Einfahren" der Säulen werden diese HPLC-Systeme bei einem Druck von ca. 90 bar und einer Flußrate von 1,8 ml/min für 60 min. gespült. Als mobile Phase dient ein wäßriges Puffersystem pH 7,4 (Puffer A). Bei allen weiteren Experimenten wird standardmäßig bei 23°C chromatographiert, der Druck beträgt 90 bar, die Flußrate 1,8 ml/min. Unter diesen Bedingungen liegen die Lipide in den Membranen A1 und A2 in der fluiden Phase vor.

Zur Bestimmung der Totzeit t0 im gewählten System wird 5 µl einer 5 mM Kaliumiodidlösung in Puffer A injiziert. Im Falle von HPLC-Säule A1 wird reproduzierbar ein t0 von 0,64 min gemessen, für Säule A2 wurde t0 = 0,58 min. bestimmt.

Anschließend werden die Säulen zur Kalibrierung mit 8 verschiedenen Referenzsubstanzen bekannten P_{ow}s im Wertebereich 1 - 4 beschickt (Acetanilid, Benzonitril, Phenol, Nitrobenzol, Benzol, Anisol, Toluol und Chlorbenzol). Bei der Auswahl der Referenzsubstanzen sollte darauf geachtet werden, daß alle Verbindungen bei den gewählten pH-Bedingungen für die mobile Phase vollständig im nicht-ionisierten Zustand vorliegen. Für diesen Fall ist davon auszugehen, daß das Verteilungsgleichgewicht zwischen Oktanol und Wasser äußerst exakt auch die Membran-Wasser-Verteilung beschreibt, da unter diesen Bedingungen elektrostatische Wechselwirkungen zwischen Testsubstanz und Sorptionsfläche vernachlässigt werden können.

Die jeweiligen Mengen an einzuspritzender Verbindung werden dabei einerseits von dessen Adsorptionstendenz an die stationäre Phase bestimmt, anderseits von der Detektierbarkeit der Verbindung. In der Regel können die meisten Referenzverbindungen mit Hilfe spektroskopischer Methoden (hier UV/Vis-Spektroskopie bei 210 nm) detektiert werden. Die gemessenen Retentionszeiten tR werden über ein Gleichungssytem nach Stand der Technik in direkte Korrelation mit dem oktanol/Wasser Verteilungskoeffizienten gesetzt. Die lineare Abhängigkeit erlaubt die Berechnung von Regressionsgeraden.

Das "Eichchromatogramm" der acht Referenzsubstanzen kann nach Identifizierung der Einzelpeaks sehr gut im Gemisch aufgenommen werden. Die erhaltene Kurve ist bei 20 aufeinanderfolgenden Chromatographieläufen mit den Säulen A1 und A2 exakt reproduzierbar. Die gemessenen Standardabweichungen für tR sind stets kleiner als 0,2 min.

### 6. Bestimmung der Membran-Wasser-Verteilungsgleichgewichte von pharmazeutisch relevanten Verbindungen

Pharmazeutisch relevante Verbindungen verfügen meistens über mindestens eine ionisierbare Funktion. Die gemessenen Verteilunskoeffizienten gelten demnach genau für den jeweiligen definierten pH-Wert unter Meßbedingungen und werden durch einen bloßen Oktanol/Wasser-Verteilungskoeffizienten nur unzureichend wiedergegeben. Verteilungsgleichgewichte ionisierter Verbindungen werden im Unterschied zu denen neutraler Verbindungen nicht über logP-Werte, sondern über die pH-abhängigen logD-Werte quantifiziert. In den vorliegenden Beispiel wird konkret jeweils logD_{Ei-PC}, pH 7.4 bestimmt. Der ß-Blocker Acetbutolol, sowie das Rattengift Warfarin (3-( -Acetonylbenzyl)-4-hydroxycoumarin) werden jeweils in einer Konzentration von 1 mg/ml in Puffer A gelöst und jeweils 5 µl in die Säulen A1 und A2 injiziert.

Für Acetbutolol wird in drei aufeinanderfolgenden Messungen mit Säule A1 reproduzierbar eine Retentionszeit von tR = 4,0 min gemessen. Aus der Kalibrierungskurve (siehe 5.) errechnet sich ein zugehöriger Verteilungskoeffizient von logD_{Ei-PC}, pH 7.4 = 2,2. Messungen mit der Säule A2 geben innerhalb eines Fehlers von 3% identische Ergebnisse. In der Literatur (Betageri, G.V., Rogers J.A., "Thermodynamics of partitioning of β-blockers in the n-octanol buffer and liposome systems, Int. J. Pharmaceutics, 36, 165-173 (1987)) wird für die Oktanol-Wasser-Verteilung von Acetbutolol bei pH 7,4 ein logD_{ow}, pH 7.4 von 2,43 angegeben, für die Anlagerung an multilamellare Lipidschichten aus Dimyristoylphosphatidylcholin bestimmen die Autoren mit äußerst zeitaufwendigen und kostenintensiven Experimenten (Ultrazentriugation) in der fluiden Phase der Lipide ein logD_{DMPC}, pH 7.4 von 2,93. Das vorgestellte Verfahren liefert somit einen korrekten Wert für das Lipid-Wasser-Verteilungsgleichgewicht, leistet dies jedoch im Gegensatz zu bisherigen Verfahren in äußerst effektiver und kostengünstiger Weise.

Analoge Aussagen zur Effizienz und Aussagekraft des vorgestellten Verfahrens liefert das Vergleichsexperiment mit Warfarin. Die HPLC-Methode mit festkörperunterstützen Membranen (Säule A1) ergibt hier reproduzierbar eine Retentionszeit von 1,2 min, das entspricht laut Kalibrierung einem logD_{Ei-PC}, pH 7.4 von 1,1. Der logD_{ow}, pH 7.4 für diese Verbindung beträgt 0,88, für unilamellare Vesikel aus Dioleoylphosphatidylcholin (deren Analytik wiederum die äußerst zeitaufwendige Ultrazentrifugation erfordert) wird laut Literaturangaben ein log_{DOPC}, pH 7.4 von 1,55 bestimmt (Avdeef, A., Box, K.J., Comer., E.A., Hibbert, C,. and K.Y. Tam, pH-Metric logP10. Determination of Liposomal Membrane-Water Partition Coefficients of ionizable Drugs, Pharmaceutical research, 15, 209-215 (1998)).

## Patentansprüche

1. Verfahren zur Untersuchung der unspezifischen Wechselwirkung von Substanzen oder Stoffen mit Oberflächen oder Grenzflächen aus amphiphilen Molekülen, die im sogenannten fluiden Zustand auf einem Träger als fester Phase derart fixiert sind, dass sie in ihrer lateralen Diffusion im wesentlichen unbeeinträchtigt sind, wobei
(a) die in einer wässrigen, mobilen Phase vorliegenden zu untersuchenden Substanzen oder Stoffe einerseits und der Träger mit den amphiphilen Molekülen andererseits in Kontakt gebracht werden,
(b) der Träger mit den amphiphilen Molekülen und den mit diesen wechselwirkenden Substanzen oder Stoffen von der mobilen Phase abgetrennt wird,
(c) die Konzentrationen der zu untersuchenden Substanzen oder Stoffe in der mobilen Phase und/oder in der Phase mit dem Träger bestimmt werden und
(d) aus den Konzentrationen die Bindungskonstanten und/oder Verteilungskoeffizienten der Substanzen oder Stoffe bestimmt werden,
wobei die amphiphilen Moleküle Lipide, Lipidderivate, lipidähnliche oder lipidanaloge Substanzen oder Gemische derartiger Substanzen sind.

2. Verfahren nach Anspruch 1, **dadurch gekennzeichnet, daß** das Verfahren mit einer definierten Menge von zu untersuchender Substanz oder Stoff und vorzugsweise mit einer definierten Menge von fester Phase mit den amphiphilen Molekülen durchgeführt wird.

3. Verfahren nach Anspruch 1 oder 2, **dadurch gekennzeichnet, daß** das Verfahren mit unterschiedlichen Mengen von zu untersuchender Substanz oder Stoff und/oder fester Phase mit den amphiphilen Molekülen wiederholt durchgeführt wird.

4. Verfahren nach einem der vorhergehenden Ansprüche, **dadurch gekennzeichnet, daß** das Verfahren unter Bedingungen durchgeführt wird, bei welchen die amphiphilen Moleküle im sogenannten fluiden Zustand bei Temperaturen oberhalb der sogenannten Phasenübergangstemperatur der amphiphilen Moleküle vorliegen.

5. Verfahren nach Anspruch 4, **dadurch gekennzeichnet, daß** die Phasenübergangstemperatur unterhalb 37°C, vorzugsweise unterhalb der Raumtemperatur liegt.

6. Verfahren nach einem der vorhergehenden Ansprüche, **dadurch gekennzeichnet, daß** die amphiphilen Moleküle durch nicht-spezifische intermolekulare Kräfte, vorzugsweise durch van-der-Waals-Kräfte oder elektrostatische Kräfte, auf dem Träger fixiert sind.

7. Verfahren nach einem der vorhergehenden Ansprüche, **dadurch gekennzeichnet, daß** die amphiphilen Moleküle in einer monomolekularen Schicht (Monolayer) angeordnet sind.

8. Verfahren nach einem der vorhergehenden Ansprüche, **dadurch gekennzeichnet, daß** die amphiphilen Moleküle in einer Doppelschicht (Bilayer) angeordnet sind.

9. Verfahren nach einem der vorhergehenden Ansprüche, **dadurch gekennzeichnet, daß** die amphiphilen Moleküle den Träger im wesentlichen vollständig umschließen.

10. Verfahren nach einem der vorhergehenden Ansprüche, **dadurch gekennzeichnet, daß** die amphiphilen Moleküle zumindest teilweise Phospholipide sind.

11. Verfahren nach einem der vorhergehenden Ansprüche, **dadurch gekennzeichnet, daß** der Träger zumindest teilweise aus kristallisierten Verbindungen, Silizium, Siliziumverbindungen, bevorzugt Silikate oder Gläser, Metallen, Metallfilmen, Aluminium, Aluminiumverbindungen, Titan, Titanverbindungen oder Polymeren besteht.

12. Verfahren nach einem der vorhergehenden Ansprüche, **dadurch gekennzeichnet, daß** der Träger ein poröses Material, insbesondere ein Gel aus porösen Silikatkügelchen ist.

13. Verfahren nach einem der vorhergehenden Ansprüche, **dadurch gekennzeichnet, daß** die zu untersuchenden Substanzen oder Stoffe Peptide, Proteine, insbesondere Enzyme, Nukleinsäuren, insbesondere DNA oder RNA, Tenside, Steroide oder Polymere sind.

14. Verfahren nach einem der vorhergehenden Ansprüche, **dadurch gekennzeichnet, daß** die zu untersuchenden Substanzen oder Stoffe pharmazeutische Wirkstoffe oder Derivate solcher Wirkstoffe sind.

15. Verfahren nach einem der vorhergehenden Ansprüche, **dadurch gekennzeichnet, daß** die mobile Phase neben den zu untersuchenden Substanzen oder Stoffen weitere Substanzen oder Stoffe enthält.

16. Verfahren nach einem der vorhergehenden Ansprüche, **dadurch gekennzeichnet, daß** die Abtrennung der mobilen Phase von der festen Phase durch Sedimentation, Zentrifugation oder Filtration erfolgt, wobei die Filtration bevorzugt ist.

17. Verfahren nach einem der vorhergehenden Ansprüche, **dadurch gekennzeichnet, daß** die Bestimmung der Konzentrationen der zu untersuchenden Substanzen oder Stoffe mit spektroskopischen, chromatographischen, chemischen, radioaktiven und/oder optischen Methoden durchgeführt wird.

18. Verwendung eines Trägers, der mit amphiphilen Molekülen in sogenannter fluider Phase derart beschichtet ist, daß die amphiphilen Moleküle in ihrer lateralen Diffusion im wesentlichen unbeeinträchtigt sind, zur Untersuchung der Lipophilität von Substanzen oder Stoffen in einem Verfahren nach einem der vorhergehenden Ansprüche, wobei die amphiphilen Moleküle Lipide, Lipidderivate, lipidähnliche oder lipidanaloge Substanzen oder Gemische derartiger Substanzen sind.

19. Kit zur Untersuchung der Lipophilität von Substanzen oder Stoffen in einem Verfahren gemäß einem der Ansprüche 1 bis 17, umfassend einen mit amphiphilen Molekülen in sogenannter fluider Phase beschichteten Träger, wobei die amphiphilen Moleküle in ihrer lateralen Diffusion im wesentlichen unbeeinträchtigt sind, und wobei die amphiphilen Moleküle Lipide, Lipidderivate, lipidähnliche oder lipidanaloge Substanzen oder Gemische derartiger Substanzen sind; und einen unbeschichteten Träger als Referenz, wobei die Träger als Suspensionen definierter Konzentration in einem vorzugsweise neutralen, wässrigen Puffer vorliegen.

20. Kit nach Anspruch 19, **dadurch gekennzeichnet, daß** der Träger ein Gel aus porösen Silikatkügelchen ist, vorzugsweise mit einer Partikelgröße von etwa 30 µm, einer Porengröße von etwa 4000 Angström und einer Oberfläche von etwa 10 m²/g.

21. Kit nach Anspruch 19 oder 20, umfassend
(a) mindestens einen mit neutralen amphiphilen Molekülen beschichteten Träger und/oder
(b) mindestens einen mit insgesamt negativ geladenen amphiphilen Molekülen beschichteten Träger.

22. Kit nach einem der Ansprüche 19 bis 21, **dadurch gekennzeichnet, daß** der Träger in einer Konzentration von etwa 0,5 g Trägermaterial pro ml Suspension vorliegt.

23. Verfahren zur säulenchromatographischen Untersuchung der unspezifischen Wechselwirkung von Substanzen oder Stoffen mit Oberflächen oder Grenzflächen aus amphiphilen Molekülen, die im sogenannten fluiden Zustand auf einem Träger als fester Phase derart fixiert sind, dass die amphiphilen Moleküle in ihrer lateralen Diffusion im wesentlichen unbeeinträchtigt sind, wobei
(a) die zu untersuchenden Substanzen oder Stoffe in einer wässrigen, mobilen Phase in die Säule, welche mit dem genannten Träger gefüllt ist, eingebracht werden,
(b) die zu untersuchenden Substanzen oder Stoffe mit dem Träger in mindestens einem Säulenchromatographie-Lauf, insbesondere einem HPLC-Lauf, in Kontakt gebracht werden,
(c) die Retentionszeiten der zu untersuchenden Substanzen bestimmt werden und
(d) aus den Retentionszeiten die Bindungskonstanten und/oder Verteilungskoeffizienten der Substanzen oder Stoffe bestimmt werden.
wobei die amphiphilen Moleküle Lipide, Lipidderivate, lipidähnliche oder lipidanaloge Substanzen oder Gemische derartiger Substanzen sind.

24. Verfahren nach Anspruch 23, **dadurch gekennzeichnet, daß** das Verfahren mit einer definierten Menge von zu untersuchender Substanz oder Stoff durchgeführt wird.

25. Verfahren nach Anspruch 23 oder 24, **dadurch gekennzeichnet, daß** das Verfahren mit unterschiedlichen Mengen von zu untersuchender Substanz oder Stoff wiederholt durchgeführt wird.

26. Verfahren nach einem der Ansprüche 23 bis 25, **dadurch gekennzeichnet, daß** das Verfahren unter Bedingungen durchgeführt wird, bei welchen die amphiphilen Moleküle im sogenannten fluiden Zustand bei Temperaturen oberhalb der sogenannten Phasenübergangstemperatur der amphiphilen Moleküle vorliegen.

27. Verfahren nach Anspruch 26, **dadurch gekennzeichnet, daß** die Phasenübergangstemperatur unterhalb 37°C, insbesondere unterhalb der Raumtemperatur liegt.

28. Verfahren nach einem der Ansprüche 23 bis 27, **dadurch gekennzeichnet, daß** die amphiphilen Moleküle durch nicht-spezifische intermolekulare Kräfte, vorzugsweise durch van-der-Waals-Kräfte oder elektrostatische Kräfte, auf dem Träger fixiert sind.

29. Verfahren nach einem der Ansprüche 23 bis 28, **dadurch gekennzeichnet, daß** die amphiphilen Moleküle in einer monomolekularen Schicht (Monolayer) angeordnet sind.

30. Verfahren nach einem der Ansprüche 23 bis 29, **dadurch gekennzeichnet, daß** die amphiphilen Moleküle in einer Doppelschicht (Bilayer) angeordnet sind.

31. Verfahren nach einem der Ansprüche 23 bis 30, **dadurch gekennzeichnet, daß** die amphiphilen Moleküle den Träger im wesentlichen vollständig umschließen.

32. Verfahren nach einem der Ansprüche 23 bis 31, **dadurch gekennzeichnet, daß** die amphiphilen Moleküle zumindest teilweise Phospholipide sind.

33. Verfahren nach einem der Ansprüche 23 bis 32, **dadurch gekennzeichnet, daß** der Träger zumindest teilweise aus kristallisierten Verbindungen, Silizium, Siliziumverbindungen, bevorzugt Silikate oder Gläser, Metallen, Metallfilmen, Aluminium, Aluminiumverbindungen, Titan, Titanverbindungen oder Polymeren besteht.

34. Verfahren nach einem der Ansprüche 23 bis 33, **dadurch gekennzeichnet, daß** der Träger ein poröses Material, insbesondere ein Gel aus porösen Silikatkügelchen ist.

35. Verfahren nach einem der Ansprüche 23 bis 34, **dadurch gekennzeichnet, daß** der Träger ein Material für die Säulenchromatographie, insbesondere für die Hochdrucksäulenchromatographie, ist.

36. Verfahren nach einem der Ansprüche 23 bis 35, **dadurch gekennzeichnet, daß** die zu untersuchenden Substanzen oder Stoffe - Peptide oder Proteine, insbesondere Enzyme, Nukleinsäuren, insbesondere DNA oder RNA, Tenside, Steroide oder Polymere sind.

37. Verfahren nach einem der Ansprüche 23 bis 36, **dadurch gekennzeichnet, daß** die zu untersuchenden Substanzen oder Stoffe pharmazeutische Wirkstoffe oder Derivate solcher Wirkstoffe sind.

38. Verfahren nach einem der Ansprüche 23 bis 37, **dadurch gekennzeichnet, daß** die mobile Phase neben den zu untersuchenden Substanzen oder Stoffen weitere Substanzen oder Stoffe enthält.

39. Verwendung eines Trägers, der mit amphiphilen Molekülen in sogenannter fluider Phase derart beschichtet ist, daß die amphiphilen Moleküle in ihrer lateralen Diffusion im wesentlichen unbeeinträchtigt sind, zur Untersuchung der Lipophilität von Substanzen oder Stoffen in der Säulenchromatographie, insbesondere der Hochdruckflüssigkeitschromatographie in einem Verfahren nach einem der Ansprüche 23 bis 38,
wobei die amphiphilen Moleküle Lipide, Lipidderivate, lipidähnliche oder lipidanaloge Substanzen oder Gemische derartiger Substanzen sind.

## Claims

1. Process for analysis of the unspecific interaction of substances or materials with surfaces or interfaces made from amphiphilic molecules which are fixed in the fluid state to a carrier as stationary phase such that they are essentially unimpeded in their lateral diffusion, wherein
(a) the substances or materials to be analysed, which are present in an aqueous, mobile phase, and the carrier with the amphiphilic molecules are brought into contact,
(b) the carrier with the amphiphilic molecules and the substances or materials interacting therewith is separated from the mobile phase,
(c) the concentrations of the substances or materials to be analysed in the mobile phase and/or in the phase with the carrier are determined, and
(d) the binding constants and/or partition coefficients of the substances or materials are determined from the concentrations,
wherein the amphiphilic molecules are lipids, lipid derivatives, lipid-similar or lipid-analogous substances or mixtures thereof.

2. Process according to Claim 1, **characterized in that** the process is performed with a defined amount of substance or material to be analysed and preferably with a defined amount of stationary phase with the amphiphilic molecules.

3. Process according to Claim 1 or 2, **characterized in that** the process is repeatedly performed with different amounts of substance or material to be analysed and/or stationary phase with the amphiphilic molecules.

4. Process according to any one of the preceding claims, **characterized in that** the process is performed under conditions where the amphiphilic molecules are present in the fluid state at temperatures above the phase transition temperature of the amphiphilic molecules.

5. Process according to Claim 4, **characterized in that** the phase transition temperature is below 37°C, preferably below room temperature.

6. Process according to any one of the preceding claims, **characterized in that** the amphiphilic molecules are fixed on the carrier by non-specific intermolecular forces, preferably by van-der-Waals forces or electrostatic forces.

7. Process according to any one of the preceding claims, **characterized in that** the amphiphilic molecules are arranged in a monomolecular layer (monolayer).

8. Process according to any one of the preceding claims, **characterized in that** the amphiphilic molecules are arranged in a bilayer.

9. Process according to any one of the preceding claims, **characterized in that** the amphiphilic molecules surround the carrier essentially completely.

10. Process according to any one of the preceding claims, **characterized in that** the amphiphilic molecules are at least partly phospholipids.

11. Process according to any one of the preceding claims, **characterized in that** the carrier comprises crystalline compounds, silicon, silicon compounds, preferably silicates or glasses, metals, metal films, aluminium, aluminium compounds, titanium, titanium compounds or polymers.

12. Process according to any one of the preceding claims, **characterized in that** the carrier is a porous material, in particular a gel of porous spherules of silicate.

13. Process according to any one of the preceding claims, **characterized in that** the substances or materials to be analysed are peptides, proteins, in particular enzymes, nucleic acids, in particular DNA or RNA, surfactants, steroids or polymers.

14. Process according to any one of the preceding claims, **characterized in that** the substances or materials to be analysed are pharmaceutically active materials or derivatives thereof.

15. Process according to any one of the preceding claims, **characterized in that**, as well as the substances or materials to be analysed, the mobile phase contains further substances or materials.

16. Process according to any one of the preceding claims, **characterized in that** the mobile phase is separated from the stationary phase by sedimentation, centrifugation or filtration, preferably filtration.

17. Process according to any one of the preceding claims, **characterized in that** the concentrations of the substances or materials to be analysed are determined using spectroscopic, chromatographic, chemical, radioactive and/or optical methods.

18. Use of a carrier coated with amphiphilic molecules in a fluid phase such that the amphiphilic molecules are essentially unimpeded in their lateral diffusion, for analysing the lipophilicity of substances or materials in a process according to any one of the preceding claims, wherein the amphiphilic molecules are lipids, lipid derivatives, lipid-similar or lipid-analogous substances or mixtures thereof.

19. Kit for analysing the lipophilicity of substances or materials in a process according to any one of Claims 1 to 17, comprising a carrier coated with amphiphilic molecules in a fluid phase, wherein the amphiphilic molecules are essentially unimpeded in their lateral diffusion and wherein the amphiphilic molecules are lipids, lipid derivatives, lipid-similar or lipid-analogous substances or mixtures thereof; and an uncoated carrier as reference,
wherein the carriers are present as suspensions of defined concentration in a preferably neutral, aqueous buffer.

20. Kit according to Claim 19, **characterized in that** the carrier is a gel of porous spherules of silicate, preferably having a particle size of about 30 µm, a pore size of about 4000 angström and a surface area of about 10 m²/g.

21. Kit according to Claim 19 or 20, comprising
(a) at least one carrier coated with neutral amphiphilic molecules, and/or
(b) at least one carrier coated with amphiphilic molecules having a net negative charge.

22. Kit according to any one of Claims 19 to 21, **characterized in that** the carrier is present in a concentration of about 0.5 g of carrier material per ml of suspension.

23. Process for column-chromatographic analysis of the unspecific interaction of substances or materials with surfaces or interfaces made from amphiphilic molecules which are fixed in the fluid state to a carrier as stationary phase such that they are essentially unimpeded in their lateral diffusion,
wherein
(a) the substances or materials to be analysed are introduced in an aqueous, mobile phase into the column which is packed with the said carrier,
(b) the substances or materials to be analysed are brought into contact with the carrier in at least one column chromatography run, in particular an HPLC run,
(c) the retention times of the substances to be analysed are determined, and
(d) the binding constants and/or partition coefficients of the substances or materials are determined from the retention times,
wherein the amphiphilic molecules are lipids, lipid derivatives, lipid-similar or lipid-analogous substances or mixtures thereof.

24. Process according to Claim 23, **characterized in that** the process is performed with a defined amount of substance or material to be analysed.

25. Process according to Claim 23 or 24, **characterized in that** the process is repeatedly performed with different amounts of substance or material to be analysed.

26. Process according to any one of Claims 23 to 25, **characterized in that** the process is performed under conditions where the amphiphilic molecules are present in the fluid state at temperatures above the phase transition temperature of the amphiphilic molecules.

27. Process according to Claim 26, **characterized in that** the phase transition temperature is below 37°C, preferably below room temperature.

28. Process according to any one of Claims 23 to 27, **characterized in that** the amphiphilic molecules are fixed on the carrier by non-specific intermolecular forces, preferably by van der Waals forces or electrostatic forces.

29. Process according to any one of Claims 23 to 28, **characterized in that** the amphiphilic molecules are arranged in a monomolecular layer (monolayer).

30. Process according to any one of Claims 23 to 29, **characterized in that** the amphiphilic molecules are arranged in a bilayer.

31. Process according to any one of Claims 23 to 30, **characterized in that** the amphiphilic molecules surround the carrier essentially completely.

32. Process according to any one of Claims 23 to 31, **characterized in that** the amphiphilic molecules are at least partly phospholipids.

33. Process according to any one of Claims 23 to 32, **characterized in that** the carrier comprises crystalline compounds, silicon, silicon compounds, preferably silicates or glasses, metals, metal films, aluminium, aluminium compounds, titanium, titanium compounds or polymers.

34. Process according to any one of Claims 23 to 33, **characterized in that** the carrier is a porous material, in particular a gel of porous spherules of silicate.

35. Process according to any one of Claims 23 to 34, **characterized in that** the carrier is a material for column chromatography, in particular for high pressure column chromatography.

36. Process according to any one of Claims 23 to 35, **characterized in that** the substances or materials to be analysed are peptides or proteins, in particular enzymes, nucleic acids, in particular DNA or RNA, surfactants, steroids or polymers.

37. Process according to any one of Claims 23 to 36, **characterized in that** the substances or materials to be analysed are pharmaceutically active materials or derivatives thereof.

38. Process according to any one of Claims 23 to 37, **characterized in that**, as well as the substances or materials to be analysed, the mobile phase contains further substances or materials.

39. Use of a carrier coated with amphiphilic molecules in a fluid phase such that the amphiphilic molecules are essentially unimpeded in their lateral diffusion, for analysing the lipophilicity of substances or materials in column chromatography, in particular high pressure liquid chromatography, in a process according to any one of Claims 23 to 38, wherein the amphiphilic molecules are lipids, lipid derivatives, lipid-similar or lipid-analogous substances or mixtures thereof.

## Revendications

1. Procédé pour étudier l'interaction non spécifique de substances ou de matériaux avec des surfaces ou des interfaces composées de molécules amphiphiles, qui sont fixées selon un mode que l'on désigne par état fluide sur un support comme phase solide, de sorte qu'elles ne soient sensiblement pas gênées dans leur diffusion latérale, dans lequel :
(a) on met en contact, d'une part, les substances ou matériaux que l'on souhaite étudier, qui sont présents dans une phase aqueuse mobile et, d'autre part, le support présentant les molécules amphiphiles,
(b) on sépare de la phase mobile le support présentant les molécules amphiphiles et les substances ou matériaux interagissant avec ces dernières,
(c) on détermine les concentrations des substances ou matériaux que l'on souhaite étudier, dans la phase mobile et/ou dans la phase contenant le support, et
(d) on détermine, à partir des concentrations, les constantes de liaison et/ou les coefficients de partage des substances ou des matériaux,
les molécules amphiphiles étant des lipides, des dérivés de lipides, des substances similaires ou analogues à des lipides, ou des mélanges de ces substances.

2. Procédé selon la revendication 1, **caractérisé en ce que** l'on réalise le procédé en utilisant une quantité définie de la substance ou du matériau que l'on souhaite étudier et, de préférence, avec une quantité définie de phase solide présentant les molécules amphiphiles.

3. Procédé selon la revendication 1 ou 2, **caractérisé en ce que** l'on répète le procédé en employant diverses quantités de la substance ou du matériau que l'on souhaite étudier et/ou de la phase solide présentant les molécules amphiphiles.

4. Procédé selon l'une quelconque des revendications précédentes, **caractérisé en ce que** l'on réalise le procédé dans des conditions selon lesquelles les molécules amphiphiles se retrouvent sous la forme de ce que l'on appelle l'état fluide, à des températures supérieures à ce que l'on désigne par température de transition de phase des molécules amphiphiles.

5. Procédé selon la revendication 4, **caractérisé en ce que** la température de transition de phase se situe en dessous de 37°C, de préférence, en dessous de la température ambiante.

6. Procédé selon l'une quelconque des revendications précédentes, **caractérisé en ce que** les molécules amphiphiles sont fixées au support par le biais de forces intermoléculaires non spécifiques, de préférence, par le biais de forces de van der Waals ou de forces électrostatiques.

7. Procédé selon l'une quelconque des revendications précédentes, **caractérisé en ce que** les molécules amphiphiles sont aménagées sous la forme d'une couche monomoléculaire (monocouche).

8. Procédé selon l'une quelconque des revendications précédentes, **caractérisé en ce que** les molécules amphiphiles sont aménagées sous la forme d'une double couche (bicouche).

9. Procédé selon l'une quelconque des revendications précédentes, **caractérisé en ce que** les molécules amphiphiles enveloppent sensiblement complètement le support.

10. Procédé selon l'une quelconque des revendications précédentes, **caractérisé en ce que** les molécules amphiphiles sont, au moins partiellement, des phospholipides.

11. Procédé selon l'une quelconque des revendications précédentes, **caractérisé en ce que** le support est constitué, au moins en partie, de composés cristallisés, de silicium, de composés de silicium, de préférence, de silicates ou de verre, de métaux, de films métalliques, d'aluminium, de composés d'aluminium, de titane, de composés de titane ou de polymères.

12. Procédé selon l'une quelconque des revendications précédentes, **caractérisé en ce que** le support est un matériau poreux, en particulier, un gel composé de petites sphères poreuses de silicate.

13. Procédé selon l'une quelconque des revendications précédentes, **caractérisé en ce que** les substances ou matériaux que l'on souhaite étudier, sont des peptides, des protéines, en particulier, des enzymes, des acides nucléiques, en particulier, de l'ADN ou de l'ARN, des agents tensioactifs, des stéroïdes ou des polymères.

14. Procédé selon l'une quelconque des revendications précédentes, **caractérisé en ce que** les substances ou matériaux que l'on souhaite étudier, sont des principes actifs pharmaceutiques ou des dérivés de ces principes actifs.

15. Procédé selon l'une quelconque des revendications précédentes, **caractérisé en ce que** la phase mobile contient, outre les substances ou matériaux que l'on souhaite étudier, d'autres substances ou matériaux.

16. Procédé selon l'une quelconque des revendications précédentes, **caractérisé en ce que** l'on sépare la phase mobile de la phase solide par sédimentation, centrifugation ou filtration, la filtration étant préférée.

17. Procédé selon l'une quelconque des revendications précédentes, **caractérisé en ce que** l'on effectue la détermination des concentrations des substances ou matériaux que l'on souhaite étudier, à l'aide de procédés de spectroscopie, de chromatographie, de chimie, de radioactivité et/ou d'optique.

18. Utilisation d'un support, qui est revêtu de molécules amphiphiles sous la forme de ce que l'on appelle une phase fluide, de sorte que les molécules amphiphiles ne soient sensiblement pas gênées dans leur diffusion latérale, en vue d'étudier le caractère lipophile de substances ou de matériaux dans un procédé selon l'une quelconque des revendications précédentes, les molécules amphiphiles étant des lipides, des dérivés de lipides, des substances similaires ou analogues à des lipides, ou des mélanges de ces substances.

19. Trousse pour étudier le caractère lipophile de substances ou de matériaux dans un procédé selon l'une quelconque des revendications 1 à 17, comprenant un support revêtu de molécules amphiphiles sous la forme de ce que l'on appelle une phase fluide, les molécules amphiphiles n'étant sensiblement pas gênées dans leur diffusion latérale, et les molécules amphiphiles étant des lipides, des dérivés de lipides, des substances similaires ou analogues à des lipides, ou des mélanges de ces substances ; et un support non revêtu comme référence, les supports étant présents sous la forme de suspensions de concentration définie dans un tampon aqueux, de préférence, neutre.

20. Trousse selon la revendication 19, **caractérisée en ce que** le support est un gel composé de petites sphères poreuses de silicate, de préférence, ayant une taille particulaire d'environ 30 µm, une taille de pores d'environ 4000 angströms et une surface d'environ 10 m²/g.

21. Trousse selon la revendication 19 ou 20, comprenant:
(a) au moins un support revêtu de molécules amphiphiles neutres, et/ou
(b) au moins un support revêtu de molécules amphiphiles globalement chargées négativement.

22. Trousse selon l'une quelconque des revendications 19 à 21, **caractérisée en ce que** le support se présente en concentration d'environ 0,5 g de matériau de support par ml de suspension.

23. Procédé pour étudier par chromatographie sur colonne l'interaction non spécifique de substances ou de matériaux avec des surfaces ou des interfaces composées de molécules amphiphiles, qui sont fixées selon un mode que l'on désigne par état fluide sur un support comme phase solide, de sorte qu'elles ne soient sensiblement pas gênées dans leur diffusion latérale, dans lequel:
(a) on introduit les substances ou matériaux que l'on souhaite étudier dans une phase aqueuse mobile dans la colonne, cette dernière étant remplie du support cité,
(b) on met en contact les substances ou matériaux que l'on souhaite étudier, avec le support, en effectuant au moins un passage chromatographique sur colonne, en particulier, un passage en HPLC,
(c) on détermine les temps de rétention des substances que l'on souhaite étudier, et
(d) on détermine, à partir des temps de rétention, les constantes de liaison et/ou les coefficients de partage des substances ou des matériaux,
les molécules amphiphiles étant des lipides, des dérivés de lipides, des substances similaires ou analogues à des lipides, ou des mélanges de ces substances.

24. Procédé selon la revendication 23, **caractérisé en ce que** l'on réalise le procédé avec une quantité définie de substance ou de matériau que l'on souhaite étudier.

25. Procédé selon la revendication 23 ou 24, **caractérisé en ce que** l'on répète le procédé avec diverses quantités de la substance ou du matériau que l'on souhaite étudier.

26. Procédé selon l'une quelconque des revendications 23 à 25, **caractérisé en ce que** l'on réalise le procédé dans des conditions selon lesquelles les molécules amphiphiles sont présentes sous la forme de ce que l'on appelle l'état fluide, à des températures supérieures à ce que l'on désigne par température de transition de phase des molécules amphiphiles.

27. Procédé selon la revendication 26, **caractérisé en ce que** la température de transition de phase se situe en dessous de 37°C, de préférence, en dessous de la température ambiante.

28. Procédé selon l'une quelconque des revendications 23 à 27, **caractérisé en ce que** les molécules amphiphiles sont fixées sur le support par le biais de forces intermoléculaires non spécifiques, de préférence, par le biais de forces de van der Waals ou de forces électrostatiques.

29. Procédé selon l'une quelconque des revendications 23 à 28, **caractérisé en ce que** les molécules amphiphiles sont aménagées sous la forme d'une couche monomoléculaire (monocouche).

30. Procédé selon l'une quelconque des revendications 23 à 29, **caractérisé en ce que** les molécules amphiphiles sont aménagées sous la forme d'une double couche (bicouche).

31. Procédé selon l'une quelconque des revendications 23 à 30, **caractérisé en ce que** les molécules amphiphiles enveloppent sensiblement complètement le support.

32. Procédé selon l'une quelconque des revendications 23 à 31, **caractérisé en ce que** les molécules amphiphiles sont, au moins partiellement, des phospholipides.

33. Procédé selon l'une quelconque des revendications 23 à 32, **caractérisé en ce que** le support est constitué, au moins en partie, de composés cristallisés, de silicium, de composés de silicium, de préférence, de silicates ou de verre, de métaux, de films métalliques, d'aluminium, de composés d'aluminium, de titane, de composés de titane ou de polymères.

34. Procédé selon l'une quelconque des revendications 23 à 33, **caractérisé en ce que** le support est un matériau poreux, en particulier, un gel composé de petites sphères poreuses de silicate.

35. Procédé selon l'une quelconque des revendications 23 à 34, **caractérisé en ce que** le support est un matériau convenant à la chromatographie sur colonne, en particulier, à la chromatographie sur colonne sous haute pression.

36. Procédé selon l'une quelconque des revendications 23 à 35, **caractérisé en ce que** les substances ou matériaux que l'on souhaite étudier, sont des peptides ou des protéines, en particulier, des enzymes, des acides nucléiques, en particulier, de l'ADN ou de l'ARN, des agents tensioactifs, des stéroïdes ou des polymères.

37. Procédé selon l'une quelconque des revendications 23 à 36, **caractérisé en ce que** les substances ou matériaux que l'on souhaite étudier, sont des principes actifs pharmaceutiques ou des dérivés de ces principes actifs.

38. Procédé selon l'une quelconque des revendications 23 à 37, **caractérisé en ce que** la phase mobile contient, outre les substances ou matériaux que l'on souhaite étudier, d'autres substances ou matériaux.

39. Utilisation d'un support qui est revêtu de molécules amphiphiles sous la forme de ce que l'on appelle l'état fluide, de sorte que les molécules amphiphiles ne soient sensiblement pas gênées dans leur diffusion latérale, dans le but d'étudier le caractère lipophile de substances ou de matériaux par chromatographie sur colonne, en particulier, par chromatographie sur colonne sous haute pression, dans un procédé selon l'une quelconque des revendications 23 à 38, les molécules amphiphiles étant des lipides, des dérivés de lipides, des substances similaires ou analogues à des lipides, ou des mélanges de ces substances.
